# EUROPEAN PATENT SPECIFICATION

(11) **EP 4 390 398 B1**
(45) Date of publication and mention of the grant of the patent: **23.07.2025**
(21) Application number: 22216488.1
(22) Date of filing: 23.12.2022
(51) Int. Cl.: G01N 33/68, C07K 14/00

(54) **SERUM PEPTIDE PATTERNS FOR DIAGNOSTICS OF INTRACRANIAL HEMORRHAGIC STROKE IN HUMANS AND DIFFERENTIAL DIAGNOSIS OF INTRACRANIAL HEMORRHAGIC STROKE FROM ACUTE ISCHEMIC STROKE**
SERUMPEPTIDMUSTER ZUR DIAGNOSE VON INTRAKRANIALEM HÄMORRHAGISCHEM SCHLAGANFALL BEI MENSCHEN UND DIFFERENTIALDIAGNOSE VON INTRAKRANIALEM HÄMORRHAGISCHEM SCHLAGANFALL AUS AKUTEM ISCHÄMISCHEM SCHLAGANFALL
MOTIFS PEPTIDIQUES SÉRIQUES POUR LE DIAGNOSTIC D'UN ACCIDENT VASCULAIRE CÉRÉBRAL HÉMORRAGIQUE INTRACRÂNIENS CHEZ L'HOMME ET LE DIAGNOSTIC DIFFÉRENTIEL D'UN ACCIDENT VASCULAIRE CÉRÉBRAL HÉMORRAGIQUE INTRACRÂNIEN

(43) Date of publication of application: 26.06.2024
(73) Proprietor: Uniwersytet Gdanski, 80-309 Gdansk (PL)
(72) Inventor: Kote, Sachin, 80-116 Gdansk (PL); Marek-Trzonkowska, Natalia, 80-752 Gdansk (PL); Czaplewska, Paulina, 80-461 Gdansk (PL); Müller, Marc, 80-462 Poland (PL); Pirog, Artur, 80-312 Gdansk (PL); Faktor, Jakub, 80-170 Gdansk (PL)
(74) Representative: Czarnik, Maciej

(56) References cited:
- EP-A1- 3 029 466
- WO-A1-2016/182855
- WO-A2-2020/229691
- MALICEK DAVID ET AL: "Proteomics-Based Approach to Identify Novel Blood Biomarker Candidates for Differentiating Intracerebral Hemorrhage From Ischemic Stroke-A Pilot Study", FRONTIERS IN NEUROLOGY, vol. 12, 17 December 2021 (2021-12-17), XP093047042, ISSN: 1664-2295, DOI: 10.3389/fneur.2021.713124

## Description

The invention refers to the serum peptide patterns for diagnostics of intracranial hemorrhagic (ICH) stroke in humans and differential diagnosis from acute ischemic stroke.

### State of the art

The invention is related to the serum peptide patterns for diagnostics of intracranial hemorrhagic (ICH) stroke in humans and differential diagnosis from acute ischemic stroke. The invention is based on the identification (qualitative analysis) and relative quantification (quantitative analysis) of unique for ICH multi-biomarker panel of serum peptides (identified based on amino acid sequences). 34 of the markers are increased 2 folds or more in sera of the patients with ICH as compared with the individuals without stroke and patients with acute ischemic stroke (quantitative analysis), while the next 16 markers are detectable only in patients with ICH, but not in individuals without stroke and with acute ischemic stroke (AIS). This serum peptide pattern can be used to diagnose and monitor ICH and patient response to the applied treatment.

As grade of the fold increase of the quantitative peptides correlates positively with their predictive significance for ICH diagnostics, we also define a serum peptide patterns 2, 3 and 4. The serum peptide pattern 2 is a list of 10 peptides which levels are increased 8 folds or more in sera of ICH patients as compared with individuals without stroke and in patients with AIS. The serum peptide pattern 3 is a list of 7 peptides which levels are increased 10 folds or more in sera of ICH patients as compared with individuals without stroke and in patients with AIS. The serum peptide pattern 4 is a list of 10 peptides which levels are increased 16 folds or more in sera of ICH patients as compared with individuals without stroke and in patients with AIS. Each of four serum peptide patterns can be used as a separate independent panel of markers for diagnostics of ICH and differentiation of ICH from AIS, as well as for monitoring ICH and patient response to the applied treatment.

Stroke is a type of cardiovascular disease (CVD) and one of the most frequent causes of death or disability in high-income countries. However, up to date, it is impossible to differentiate without magnetic resonance imaging (MRI) between ICH and AIS, while fast differential diagnostics is crucial for immediate implementation of the proper treatment that can save patient life and prevent from disability. As ICH and AIS have different pathophysiology, they require different treatment. Mechanical thrombectomy and chemical thrombolysis with recombinant tissue plasminogen activator (rtPA) are the main treatments used to achieve reperfusion in AIS. However, these treatments sometimes induce hemorrhagic stroke. The appearance of intracranial hemorrhagic (ICH) stroke is strictly connected with a higher likelihood of fatal intracranial hemorrhage and a higher risk of long-term disability [1] than in untreated patients. Consequently, identifying and separating patients with ICH from those with AIS remains the challenge to securely select the appropriate treatment and reduce the number of unfavored clinical outcomes. Fast and effective discrimination of the patients with ICH from those with AIS without the need for specialized equipment. This issue is of great importance in developing countries where the access to the specialized diagnostic equipment is limited and during emergency situations like recent SARS-CoV2 pandemics, where number of patients with stroke dramatically increased as a complication of COVID19 [2], [3] boosting the need for fast and efficient discrimination between different types of stroke.

Peptidomics targets and analyses peptides (their length, distribution, protein origin, or de-novo sequencing). Peptides are protein fragments with various functions. As they regulate multiple physiological and pathological processes, alterations in peptidome in cells and various body fluids may reflect both beneficial or deleterious phenomena. Thus, peptidome analysis in body fluids such as blood samples is a promising approach for identifying peptide biomarkers of various conditions and monitoring patient response to the applied treatment. The peptidome is considered as low molecular-weight peptides/proteins [4] (less than 15 kilo Daltons; kDa). Endogenous peptides primarily act as a messenger (cytokines, hormones, growth factors, etc.) and indicate various biological processes. Hence, these peptides can reflect the health or disease state of the person. Even though peptidomics has been introduced since the decade, the serum peptidome analysis has consistently been restricted for various reasons; a) enormous complexity because serum contain elements of all proteins produced in the body, b) physiologically status of high abundant proteins such as serum albumin, immunoglobulins are almost 90% of total serum protein by weight, hence these abundant proteins mask the detection of other proteins, especially low abundance proteins or peptides, c) simultaneously limit detection of low abundant peptides with diagnostic potential, d) unequal concentrations of low and high abundant proteins (around 95% of high abundant proteins and less than 5% presence of low abundant) in the total serum protein concentrations, e) instability of low abundant proteins or peptides (hormones or small secreted proteins or possible diagnostic or prognostic biomarkers), f) depletion steps, fractionation and precipitation methods for the removal of high abundant proteins are overwhelming that compromise the number, yield of diagnostic peptides identified and important biological information can be lost in the background noise. Hence, all previously described methods for serum peptide analysis were characterized by low numbers of identified peptides, and no quantitative methods for serum peptidome analysis were introduced.

In the past, few patents were published with methods, devices, kits aiming to tackle ICH biomarker discovery with different approaches such as protein/peptide levels and immunoassay [5], plasma enzymes levels [6], etc. In addition, several past reports focused only on single proteins or enzyme biomarkers for stroke patients. However, considering a single biomarker such protein alpha-1 antitrypsin [7], L-glutamine hydroxylamine glutamyl transferase (L-GHGT) and gamma glutamyl hydroxamate synthetase (GGHS) activity [6] for stroke might be too risky to rely on accurate diagnosis. On the contrary, our approach enables us to find multi biomarkers of naturally occurring peptides from intracranial hemorrhagic (ICH) stroke. Data from previous literature showed that the serum peptidomics approach had been used for the diagnosis of various ranges of diseases. However, the ultimate success of these previously published methods is severely limited due to the low number of identified markers. On the contrary, we provide unique serum peptide patterns for diagnostics of ICH in humans and differential diagnosis from AIS. 34 of the markers are increased 2-fold or more in sera of the patients with ICH as compared with the individuals without stroke and patients with AIS (quantitative peptides), while the next 16 markers are detectable only in patients with ICH, but not in individuals without stroke and with AIS (qualitative peptides). As grade of the fold increase of the 34 quantitative peptides corresponds with their predictive significance for ICH diagnostics, we also define a panel of 10 peptides, panel of 7 peptides and panel of 5 peptides which can be used alone for ICH diagnostics or differentiation from AIS when their fold increase is equal or higher than 8, 10 and 16, respectively as compared with serum levels in patients without stroke and patients with AIS.

### Essence of the invention - description of the invention

**The invention is disclosed in the claims.**

The invention is serum peptide patterns for diagnostics of intracranial hemorrhagic (ICH) stroke in human and differential diagnosis from acute ischemic stroke. The first serum peptide pattern is a list of 34 peptides (quantitative peptides) which levels are increased 2 folds or more in sera of the patients with ICH as assessed with relative comparison with serum samples of individuals without stroke and patients with AIS. The serum peptide pattern also comprises 16 peptides (qualitative peptides) that are detectable only in the patients with ICH but not in serum samples of individuals without stroke and patients with AIS. The 34 quantitative peptides are as follow (amino acid sequence of each peptide is given): Glu-Phe-Val-Ser-Glu-Thr-Glu-Ser-Arg-Gly-Ser-Glu; Ala-Glu-Pro-Glu-Gly-Gly-Pro-Ala-Ser-Glu-Gly-Ala-Ala-Arg-Pro-Leu-Pro-Arg, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Phe-Val-Ser-Glu-Thr-Glu-Ser-Arg-Gly-Ser-Glu-Ser-Gly, Thr-Gln-Val-Ser-Thr-Gln-Ala-Glu-Gln-Leu-Arg, Lys-Phe-Asp-Lys-Ser-Lys-Leu, Glu-Leu-Gln-Gly-Val-Val-Pro-Arg, Thr-Ser-Ser-Ser-Ala-Ala-Pro-Gin-Arg-Pro-fle, Ser-Val-Phe-Pro-Gln-Gln- Thr-Gly-Gln-Leu-Ala-Glu-Leu-Gln-Pro-Gln-Asp-Arg-Ala-Gly-Ala-Arg-Ala-Ser, Asn-Thr-Lys-Ser-Pro-Leu-Phe-Met-Gly-Lys-Val-Val-Asn-Pro-Thr-Gln-Lys, Tyr-Lys-His-Tyr-Asp-Gly-Ser-Tyr, Asp-Thr-Asn-Arg-Ala-Ser-Val-Gly-Gin-Asp-Ser-Pro-Glu-Pro-Arg, Phe-Ser-Pro-Val-Thr-Pro-Lys-Phe-Thr-Pro-Val-Ala-Ser, Asp-Ser-Gly-Glu-Gly-Asp-Phe-Leu-Ala-Glu-Gly-Gly-Gly-Val-Arg, Gln-Phe-Thr-Ser-Ser-Thr-Ser-Tyr-Asn-Arg-Gly-Asp-Ser, Arg-Ile-His-Trp-Glu-Ser-Ala-Ser, Val-Gly-Pro-Ala-Gly-Ala-Val-Gly-Pro-Arg, Asp-Arg-Asn-Leu-Pro-Ser-Asp-Ser-Gln-Asp-Leu-Gly-Gln-His-Gly, Lys-Lys-Pro-Leu-Thr-Ser-Ser-Ser-Ala-Ala-Pro-Gln-Arg-Pro-Ile-Ser-Thr-Gln, Asn-Asp-Asn-Glu-Glu-Gly-Phe-Phe, Ala-Leu- Thr-Ser-Glu-Glu-Ala-Glu-Arg-Ser-Asp-Gly-Asp-Pro-Val-Gln-Pro-Ala, His-Thr-Glu-Ala-Gln-Ile-Gin-Glu-Glu-Gly-Thr-Val, Ser-Thr-Ser-Tyr-Asn-Arg-Gly-Asp-Ser-Thr-Phe-Glu-Ser-Lys-Ser, Glu-Ile-Gln-Ile-Tyr-Lys-Lys-Leu-Arg, Lys-Glu-Ser-Ser-Ser-His-His-Pro-Gly-Ile-Ala-Glu-Phe-Pro-Ser, Thr-Pro-Leu-Gly-Gln-Gln-Gln-Pro-Ala-Pro-Arg, Tyr-Ser-Lys-Gin-Phe-Thr-Ser, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Ala-Leu-Gln-Leu-Asn-Asn-Arg-Gln-Ile, Val-Leu-Ser-Val-Leu-Lys-Gly-Glu-Glu, Ser-Leu-Val-Ile-Ser-Pro-Pro-Val-Arg-Thr-Ala-Thr-Val-Ser (Table 1)

The 16 qualitative peptides are (amino acid sequence of each peptide is given): Tyr-Val-Ser-Asp-Ala-Phe-His-Lys-Ala-Phe, Gly-Ala-Asp-Gly-Val-Pro-Gly-Lys-Asp-Gly-Pro-Arg-Gly-Pro, Pro-Gly-Lys-Asp-Gly-Pro-Arg-Gly-Pro-Thr, Ser-Leu-Gly-Ser-Pro-Ser-Gly-Glu-Val, Ser-Asp-Leu-Gln-Ala-Gln-Ser-Lys-Gly-Asn-Pro-Glu-Gln-Thr-Pro-Val, Asp-Arg-Lys-Tyr-Glu-Glu-Val, Leu-Gln-Arg-Val-Lys-Arg-Leu-Pro-Leu-Glu-Tyr-Ser-Tyr, Ser-Glu-Gly-Ala-Ala-Arg-Pro-Leu-Pro-Arg, Ser-Lys-Gly-Ala-Gly-Gly-Gln-Gly-Lys-Val-Ala-Ser-Lys-Ile-Val-Gly-Pro-Ser-Gly, Glu-Glu-Glu-Leu-Val-Lys-Val-Arg-Glu-Lys-Gln-Leu-Ala, Glu-Glu-Gln-Leu-Glu-Glu-Glu-Glu-Ser-Ala-Arg-Gln, Ala-Gln-Ser-Gly-Pro-Val-Arg-Ser-Gly-Pro-Lys-Pro-Phe-Ser-Ala-Pro-Lys-Pro-Gln, Ala-Gln-Ser-Gly-Pro-Val-Arg-Ser-Gly-Pro-Lys-Pro-Phe-Ser-Ala-Pro-Lys-Pro-Gln-Thr, Ala-Leu-Glu-Asn-His-Gly-Phe, Gly-Gly-Thr-Gly-Gly-Ile-Gly-Gly-Val-Gly-Gly-Thr-Gly-Gly-Val-Gly-Asn-Arg-Ala-Pro-Arg, Val-Gly-Gly-Thr-Gly-Gly-Ile-Gly-Gly-Val-Gly-Gly-Thr-Gly-Gly-Val-Gly-Asn-Arg-Ala-Pro-Arg (Table 1).

Grade of the fold increase of the quantitative peptides correlates positively with their predictive significance for ICH diagnostics. Therefore, we also define a second serum peptide pattern that is a list of 10 peptides selected from 34 quantitative peptides that can be used as a separate independent panel of markers for diagnostics of ICH and differentiation of ICH from AIS when their levels are increased 8 folds or more in sera of ICH patients as compared with individuals without stroke and in patients with AIS. These peptides are: Glu-Phe-Val-Ser-Glu-Thr-Glu-Ser-Arg-Gly-Ser-Glu, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Ala-Leu-Thr-Ser-Glu-Glu-Ala-Glu-Arg-Ser-Asp-Gly-Asp-Pro-Val-Gln-Pro-Ala, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Val-Leu-Ser-Val-Leu-Lys-Gly-Glu-Glu (Table 2).

In addition, we define a third serum peptide pattern that is a list of 7 peptides selected from 34 quantitative peptides that can be used as a separate independent panel of markers for diagnostics of ICH and differentiation of ICH from AIS when their levels are increased 10 folds or more in sera of ICH patients as compared with individuals without stroke and in patients with AIS. These peptides are: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Lys-Asn-Pro-Leu-Pro-Ser-Lys-Glu-Thr,Ser-Tyr-Ser-Lys-Gln-Phe-Thr-Ser-Ser-Thr-Ser-Tyr-Asn-Arg-Gly (Table 3).

Finally, we define a fourth serum peptide pattern that is a list of 5 peptides selected from 34 quantitative peptides that can be used as a separate independent panel of markers for diagnostics of ICH and differentiation of ICH from AIS when their levels are increased 16 folds or more in sera of ICH patients as compared with individuals without stroke and in patients with AIS. These peptides are: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Arg-Ile-His-Trp-Glu-Ser-Ala-Ser, Ser-Tyr-Ser-Lys-Gln-Phe-Thr-Ser-Ser-Thr-Ser-Tyr-Asn-Arg-Gly (Table 4).

The serum peptide patterns for diagnostics of ICH are based on qualitative and quantitative analysis of serum peptides with tandem mass spectrometry. However, the method for peptide identification and quantification is not the limitation of the invention. The same peptide patterns can be detected and measured with different techniques for peptide identification and used for ICH diagnostics and monitoring. All 4 serum peptide patters were selected during the research - approach to provide the diagnostic methods for ICH. The peptides listed in table 1 (all peptides, 50) were checked in the serum samples - confirmed during provision of the invention - approach with the adjusted p value ≤ 0.05 confirming statistically significance.

For all 34 quantitative peptides a MS/MS (tandem mass spectrometry) relative signaling intensity measurement is defined as fold change. The fold change is obtained after comparison of the signal intensity of quantitative peptides in serum of patients with ICH with signal intensity of quantitative peptides in serum of matched control individuals that are individuals without stroke and patients with AIS.

Fold-increase values provided in the table 5 for peptides 1-34 set how many times more the peptide MS/MS (tandem mass spectrometry) signaling intensities present in the serum of ICH patients was higher than in serum samples from individuals without stroke and the patients with the AIS. The serum peptide patterns for diagnostics of intracranial hemorrhagic (ICH) stroke in humans and differential diagnosis from acute ischemic stroke that are the matter of the current invention were defined and analyzed with tandem mass spectrometry. However, the method of peptide identification and quantification is not the limitation of the invention and can be different than tandem mass spectrometry. In the example provided in this patent application serum samples designated for peptide identification and quantification were treated as follow:
I. Tested serum sample preparation according to which the following steps are performed:
   a) enrichment of peptides in the analyzed serum sample by incubation of the sample with a citrate-phosphate buffer with pH 3.1-3.6 in water with a purity ≥ 99.9%, wherein the buffer and serum volume ratio is 29:1 and the buffer and serum are mixed at a temperature of range 4 to 8 °C to dissociate peptides from highly concentrated serum proteins;
   b) removing of proteins equal or bigger than 15kDa from peptide enriched and simultaneously purification of peptides using hydrophilic-lipophilic balanced columns to obtain peptides smaller than 15 kDa;
   c) washing the hydrophilic-lipophilic balanced columns with high-grade water purity ≥ 99.9% in order to remove the unbound and low-binding peptides from the columns;
   d) eluting of serum peptides bound to the hydrophilic-lipophilic balanced columns by using water/80% methanol/0.2% formic acid volume per volume (v/v);
   e) filtrating of the sample through 3kDa molecular weight filters to collect peptides smaller than 3kDa;
   f) providing lyophilization of the obtained peptides by drying the eluted peptides through lyophilization for tandem mass spectrometry analysis.

With the applied technique the diagnostic peptides used for detection of ICH were separated and purified in a single step highly concentrated serum proteins > 16 to 170 kilodaltons (kDa) (e.g. albumin, globulin, transferrin, haptoglobin, alpha-1-antitrypsin, etc.)

Then, peptides were analyzed for amino acid sequences with a tandem mass spectrometry. The data analysis workflow for quantitative analysis of the peptides sequenced with a tandem mass spectrometry comprised the following steps within the Skyline, MSStats pipeline:
a). the MS/MS DDA (data-dependent acquisition) data were searched in more than 1 search engine strategy, resulting in creation of a comprehensive spectral library;
b). the comprehensive spectral library was used to extract quantitative peptidomics data (independent MS/MS data; DIA);
c). extraction of the product ion chromatograms and integration of the product ion peak group areas (area under the curve, AUC);
d). the false discovery rate (FDR) of quantitative extraction/analysis was controlled by determining peak group's FDR values in the mProphet module;
e). performing the inference of product ion peak areas to peptides and their summed peak area statistics in MSstats module was done in a peptide-centric view;
f). further downstream analyses such as graphical data visualization including volcano plots, dendrograms, heatmaps, functional stroke serum peptide enrichment maps were done using quantitative matrix from MSstats. Thus, the downstream analyses is not the limitation of the method. The serum peptide diagnostic patters for ICH are listed in tables 1-4.

Table 1. The serum peptide pattern 1 for diagnostics of ICH and differential diagnosis of ICH from AIS. The sequences of diagnostic peptides, as well as the source protein identifier (*) for each peptide is given. The source protein for the given peptide is the protein which contains in its structure the given peptide. The peptides derive from the source proteins that underwent various types of degradations. The fold change (***) refers only to quantitative peptides and it shows how many times more signaling intensities present in the sera of ICH patients were higher than signaling intensities in serum samples from individuals without stroke and the patients with AIS.

One to 34 quantitative peptides (serial numbers 1-34) with fold change equal or higher than 2 (fold change ≥ 2) have diagnostic value and confirms the diagnosis of ICH. In case of qualitative peptides- detection of 1 to 16 of qualitative peptides (serial numbers 35-50) the peptide confirms the diagnosis of ICH.

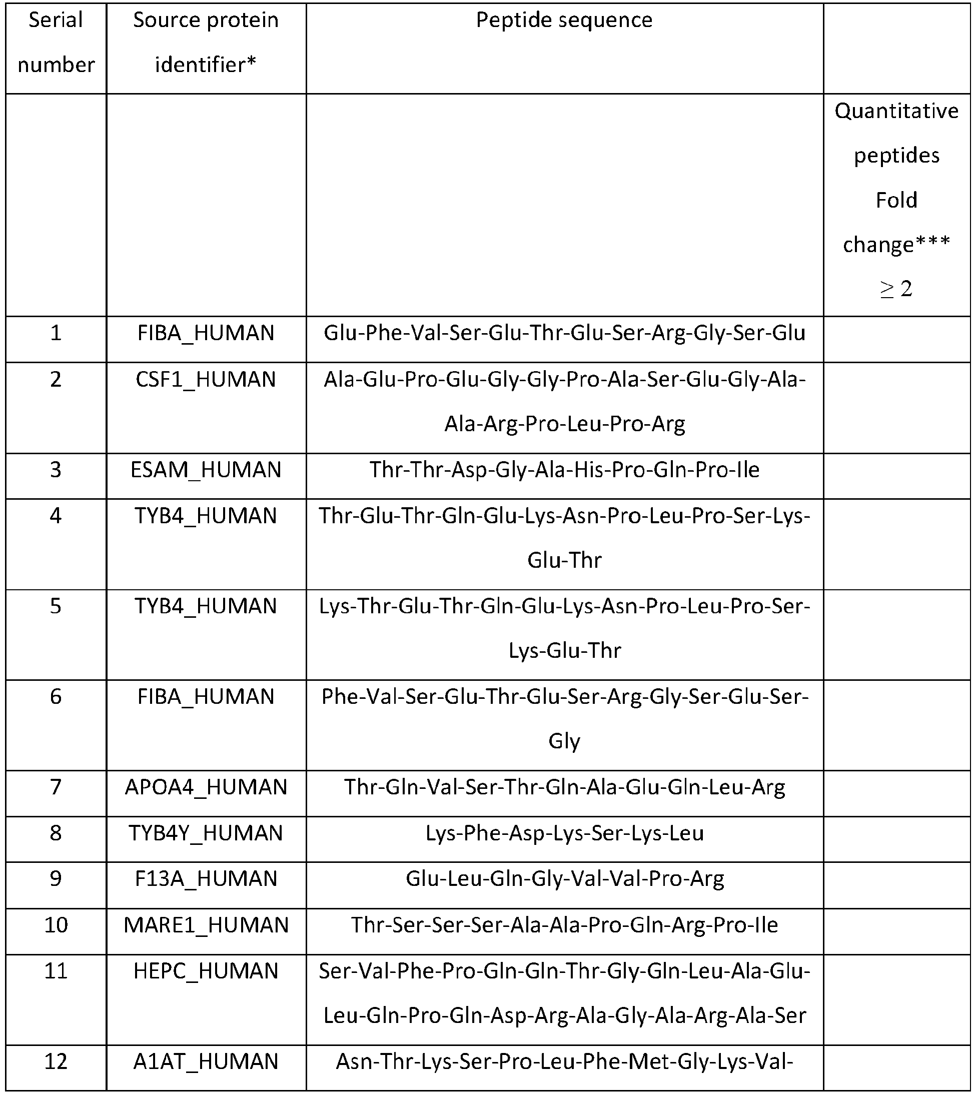

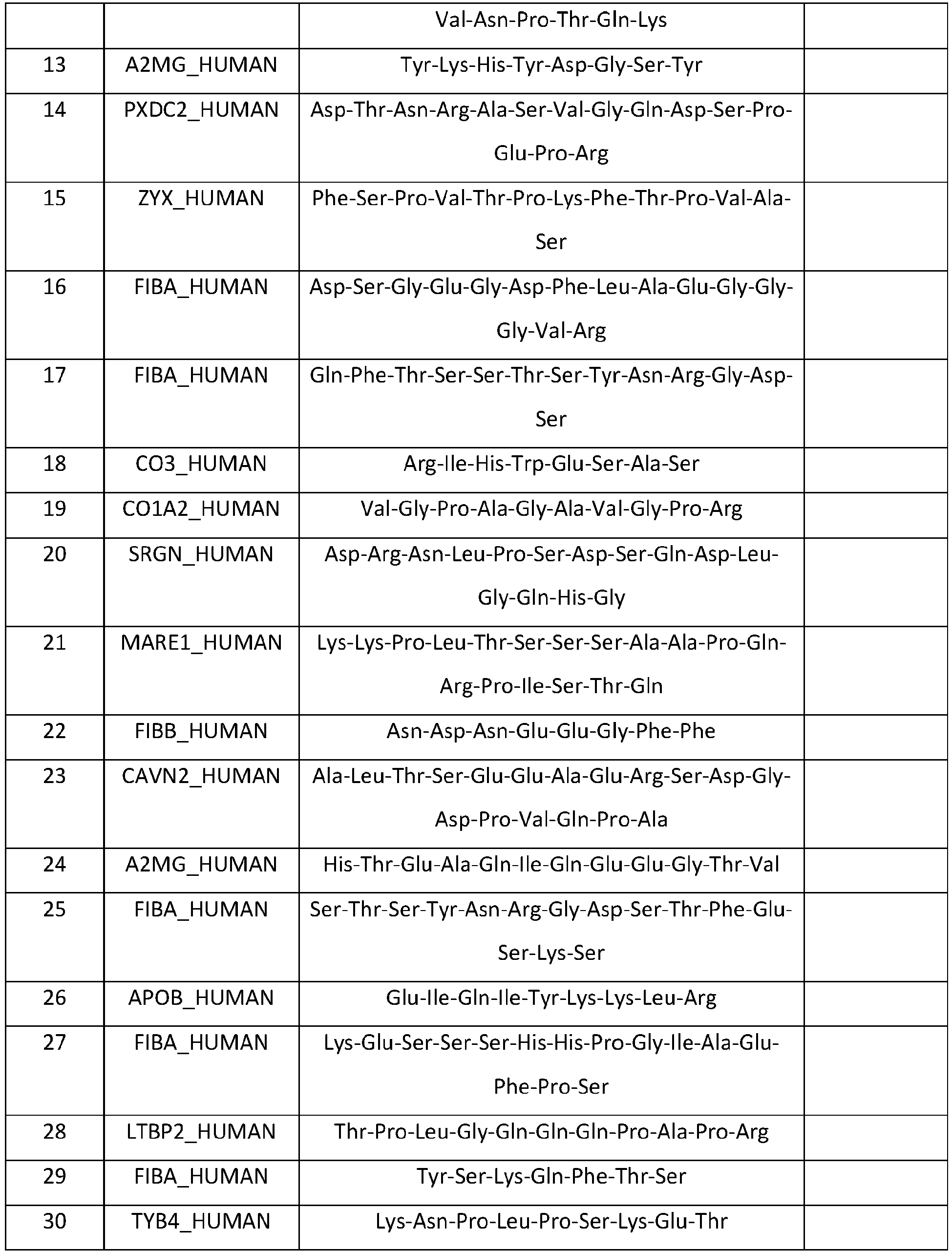

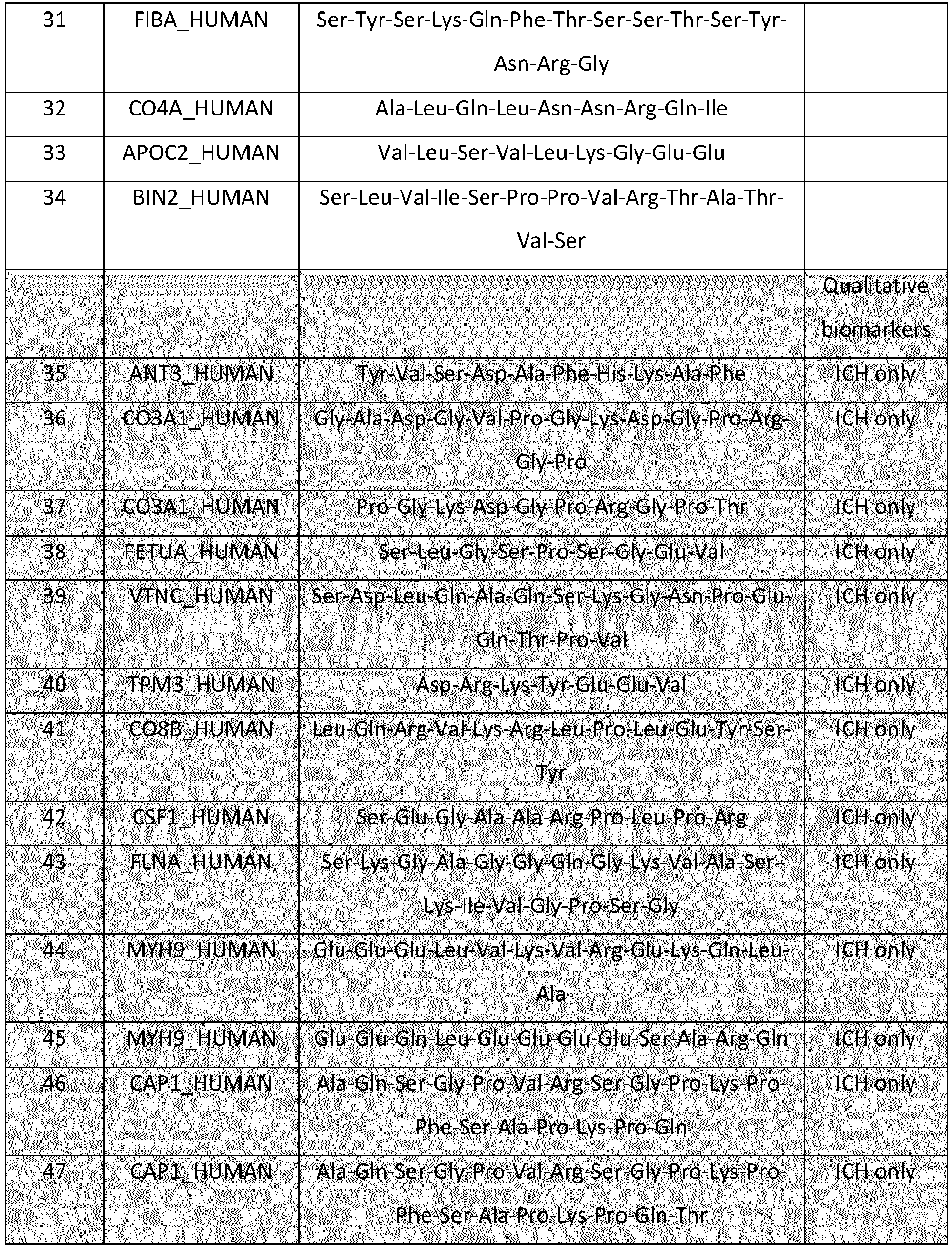

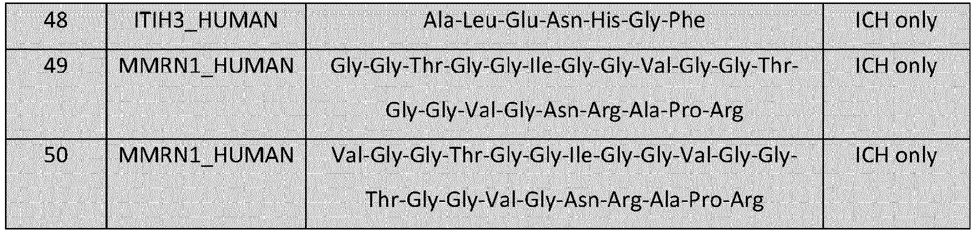

**Table 2. The serum peptide pattern 2 for diagnostics of ICH and differential diagnosis of ICH from AIS. The sequences of diagnostic peptides, as well as the source protein identifier (*) for each peptide is given. The source protein for the given peptide is the protein which contains in its structure the given peptide. The peptides derive from the source proteins that underwent various types of degradations. The fold change (***) refers only to quantitative peptides and it shows how many times more signaling intensities present in the sera of ICH patients were higher than signaling intensities in serum samples from individuals without stroke and the patients with AIS. The listed quantitative peptides with fold change equal or higher than 8 (fold change ≥ 8) have diagnostic value and confirms the diagnosis of ICH.**

| Serial number | Source protein identifier* | Peptide sequence | |
|---|---|---|---|
| | | | Quantitative peptides Fold change*** ≥ 8 |
| 1. | FIBA_HUMAN | Glu-Phe-Val-Ser-Glu-Thr-Glu-Ser-Arg-Gly-Ser-Glu | |
| 2. | ESAM_HUMAN | Thr-Thr-Asp-Gly-Ala-His-Pro-Gln-Pro-Ile | |
| 3. | TYB4_HUMAN | Thr-Glu-Thr-Gln-Glu-Lys-Asn-Pro-Leu-Pro-Ser-Lys- Glu-Thr | |
| 4. | TYB4_HUMAN | Lys-Thr-Glu-Thr-Gln-Glu-Lys-Asn-Pro-Leu-Pro-Ser- | |
| | | Lys-Glu-Thr | |
| 5. | PXDC2_HUMAN | Asp-Thr-Asn-Arg-Ala-Ser-Val-Gly-Gln-Asp-Ser-Pro- Glu-Pro-Arg | |
| 6. | CO3_HUMAN | Arg-Ile-His-Trp-Glu-Ser-Ala-Ser | |
| 7. | CAVN2_HUMAN | Ala-Leu-Thr-Ser-Glu-Glu-Ala-Glu-Arg-Ser-Asp-Gly- Asp-Pro-Val-Gln-Pro-Ala | |
| 8. | TYB4_HUMAN | Lys-Asn-Pro-Leu-Pro-Ser-Lys-Glu-Thr | |
| 9. | FIBA_HUMAN | Ser-Tyr-Ser-Lys-Gln-Phe-Thr-Ser-Ser-Thr-Ser-Tyr- Asn-Arg-Gly | |
| 10. | APOC2_HUMAN | Val-Leu-Ser-Val-Leu-Lys-Gly-Glu-Glu | |

**Table 3. The serum peptide pattern 3 for diagnostics of ICH and differential diagnosis of ICH from AIS. The sequences of diagnostic peptides, as well as the source protein identifier (*) for each peptide is given. The source protein for the given peptide is the protein which contains in its structure the given peptide. The peptides derive from the source proteins that underwent various types of degradations. The fold change (***) refers only to quantitative peptides and it shows how many times more signaling intensities present in the sera of ICH patients were higher than signaling intensities in serum samples from individuals without stroke and the patients with AIS. The listed quantitative peptides with fold change equal or higher than 10 (fold change ≥ 10) have diagnostic value and confirms the diagnosis of ICH.**

| Serial number | Source protein identifier* | Peptide sequence | |
|---|---|---|---|
| | | | Quantitative peptides Fold change*** ≥ 10 |
| 1. | FIBA_HUMAN | Glu-Phe-Val-Ser-Glu-Thr-Glu-Ser-Arg-Gly-Ser-Glu | |
| 2. | TYB4_HUMAN | Thr-Glu-Thr-Gln-Glu-Lys-Asn-Pro-Leu-Pro-Ser-Lys- Glu-Thr | |
| 3. | TYB4_HUMAN | Lys-Thr-Glu-Thr-Gln-Glu-Lys-Asn-Pro-Leu-Pro-Ser- Lys-Glu-Thr | |
| 4. | PXDC2_HUMAN | Asp-Thr-Asn-Arg-Ala-Ser-Val-Gly-Gln-Asp-Ser-Pro- Glu-Pro-Arg | |
| 5. | CO3_HUMAN | Arg-Ile-His-Trp-Glu-Ser-Ala-Ser | |
| 6. | TYB4_HUMAN | Lys-Asn-Pro-Leu-Pro-Ser-Lys-Glu-Thr | |
| 7. | FIBA_HUMAN | Ser-Tyr-Ser-Lys-Gln-Phe-Thr-Ser-Ser-Thr-Ser-Tyr- Asn-Arg-Gly | |

**Table 4. The serum peptide pattern 4 for diagnostics of ICH and differential diagnosis of ICH from AIS. The sequences of diagnostic peptides, as well as the source protein identifier (*) for each peptide is given. The source protein for the given peptide is the protein which contains in its structure the given peptide. The peptides derive from the source proteins that underwent various types of degradations. The fold change (***) refers only to quantitative peptides and it shows how many times more signaling intensities present in the sera of ICH patients were higher than signaling intensities in serum samples from individuals without stroke and the patients with AIS. The listed quantitative peptides with fold change equal or higher than 16 (fold change ≥ 16) have diagnostic value and confirms the diagnosis of ICH.**

| Serial number | Source protein identifier* | Peptide sequence | |
|---|---|---|---|
| | | | Quantitative peptides Fold change*** ≥ 16 |
| 1. | FIBA_HUMAN | Glu-Phe-Val-Ser-Glu-Thr-Glu-Ser-Arg-Gly-Ser-Glu | |
| 2. | TYB4_HUMAN | Thr-Glu-Thr-Gln-Glu-Lys-Asn-Pro-Leu-Pro-Ser-Lys- Glu-Thr | |
| 3. | TYB4_HUMAN | Lys-Thr-Glu-Thr-Gln-Glu-Lys-Asn-Pro-Leu-Pro-Ser- Lys-Glu-Thr | |
| 4. | CO3_HUMAN | Arg-Ile-His-Trp-Glu-Ser-Ala-Ser | |
| 5. | FIBA_HUMAN | Ser-Tyr-Ser-Lys-Gln-Phe-Thr-Ser-Ser-Thr-Ser-Tyr- Asn-Arg-Gly | |

Essence of the invention in detailed and the study description - approach leading to provision of the invention.

The invention differs from available approaches as we elaborated 4 complexed serum peptide patters for diagnostics of ICH and differentiation it from AIS. The first serum peptide pattern comprises of 34 quantitative and 16 qualitative peptides (Table 1), the second serum peptide pattern comprises of 10 quantitative peptides (Table 2), the third serum peptide pattern comprises of 7 quantitative peptides (Table 3), while the fourth serum peptide pattern comprises of 5 quantitative peptides (Table 4). These are the novel and not described previously serum peptides pattern that can serve for diagnostics of ICH and its differentiation from AIS.

Brief explanation of the method that provided the invention
1. Samples; a collection of serum samples from intracranial hemorrhagic (ICH) stroke patients groups. 50-100 µl of serum samples from each patient are collected and stored at -80 °C immediately until the analysis.
2. Glassware qualities; mass spectrometer is a highly sensitive analytical machine to detect the minute amount of contaminants during the sample preparation. In order to not compromise the peptides signals, it is necessary to use high grades purity of water and other chemicals.
3. Buffer preparation; unexpected use of low pH buffer for peptide release from complexes with high abundant/carrier proteins. The citrate-phosphate buffer pH 3.1-3.6 was prepared in high grade of water.
4. Incubation; peptidomics samples preparation starts with the incubation by using a glass beaker between the serum samples and an acidic buffer, citrate-phosphate buffer for 3-5 minutes (gentle and vertexing necessary) in the temperature at 4-8 °C to dissociate protein-peptide interactions especially the peptides bound to the carrier proteins like albumin. Therefore, is characterized by the unexpected use of low pH buffer for peptide release from complexes with high abundant/carrier proteins.
5. Purification of peptides; single-step purification is performed by using hydrophilic-lipophilic balanced columns. Unexpectedly this column is able to deplete the highly abundant proteins without losing the peptides and simultaneously, it purifies the peptides from other contaminants in the serum. Therefore, it allows us to yield more peptides and isolate possible carrier peptides from highly abundant proteins.
6. Elution of peptides; first several washing performed with high-grade water to remove the unbound and low-binding peptides from the columns. Then the bound serum peptides are eluted from the columns using water/80% methanol/0.2% formic acid volume per volume (v/v). Further eluted peptides collected have been passed through 3kDalton molecular weight filters. Lastly, peptides of interest are lyophilized to dryness. All dried peptide samples were stored at -80 until the mass spectrometry analysis.
7. Sequencing of peptides with tandem mass spectrometry; the dried serum peptides were further subjected to sequencing with UltiMate^{™} 3000 RSLCnano liquid chromatograph (Thermo Scientific) online coupled with Orbitrap Exploris 480 mass spectrometer (MS) (Thermo Scientific). Indexed retention time (iRT) peptides were added to each sample for controlling the retention time of serum peptides for downstream quantitative analysis. Analytical peptide separation was performed by a non-linear gradient with mobile phases A and B. Serum peptides eluting from the analytical column were ionized in a nano-electrospray ion source (NSI) connected to a mass spectrometer.
8. The isolated peptides are then sequenced based on amino acid sequences with a tandem mass spectrometry tool.

Description of the following steps of the analysis - approach leading to provision of the invention - diagnosis method.

### a) Comprehensive qualitative and quantitative analysis

The invention employed multi-search engine strategies for creating the spectral library for qualitative and quantitative serum peptidomics, facilitating to screen the diagnostic peptides for the ICH. As a result, for the first time, comprehensive qualitative serum peptides were identified. Furthermore, it enabled DDA (data-dependent acquisition) to identify a total of at least 4270 serum peptides (iprophet iprobability value > 0.99%). At the same time, at least 1900 serum peptides were successfully quantified by DIA (data-independent acquisition) LC-MS/MS (Liquid Chromatography with tandem mass spectrometry) analysis of ICH stroke samples. Therefore, high throughputs in the context of qualitative and quantitative serum peptidomics were the key for defining serum peptide patterns for ICH diagnostics, but the method that was used is not the limitation of the study.

### b) Peptide length distribution

The approach facilitates the crucial feature of serum peptidomics by screening a wide range of peptide lengths. These peptides were not generated by trypsin digestion, where the trypsin enzyme cleaves the proteins into every K (lysine) or R (arginine) site. On the contrary, the novel approach allows screening the natural and without any enzymatically digested peptides. Furthermore, the peptide length distribution is wide at 7-30 amino acid residues. Therefore, it can be easily tunable to 1-10 kilo Dalton in a range of natural serum peptides from the ICH stroke samples.

### c) Data analysis workflow

The approach deals with quantitative analysis of high throughput (≥1900 peptides quantified) serum peptidomics. The serum peptidomics quantification workflow consists of unique steps within the Skyline, MSStats pipeline. First, the MS/MS DDA (data-dependent acquisition) data are searched in a multi-search engine strategy, including for example MS-fragger, Comet search engines (different search engines can be also used). These facilitate the creation of a comprehensive spectral library in which all serum peptide identification spectral data are summarized and later used to extract quantitative peptidomics data-independent MS/MS data (DIA). Next, the skyline extracts product ion chromatograms and integrates product ion peak group areas. The false discovery rate (FDR) of analysis is controlled by determining peak groups FDR values in mProphet module. Finally, inference of product ion peak areas to peptides and their summed peak area statistics were performed in MSstats module in a peptide-centric view. Output quantitative matrix from MSstats then subjected to the downstream multi-bioinformatics analysis such as volcano plot, dendrograms, heatmaps, functional ICH stroke serum peptide enrichment maps, results shown in table 5.

9. Selection of the quantitative and qualitative peptides with the diagnostic value. The two thresholds were used for selection of the diagnostic peptides to define serum peptide patters. For the first serum peptide pattern (listed in Table 1 and 5) detected quantitative and qualitative peptides had to show adjusted P value (adj. pval) ≤ 0.05 (adjusted p value ≤0.05 is marked with "**" in table 5). In addition, in case of quantitative peptides the fold increase in serum peptide levels equal to 2 or higher was used as the second threshold for selection of diagnostic peptide to define serum peptide pattern. In table 5, the fold increase of the peptide levels is equal or higher than 2 (fold change ≥ 2) and is marked with "***" in the table 5, confirming the diagnosis of ICH.

For the second serum peptide pattern (listed in Table 2 and 6) detected quantitative peptides had to show adjusted P value (adj. pval) ≤ 0.05 (adjusted p value ≤ 0.05 is marked with "**" in table 6). The second threshold for defining the second diagnostic peptide pattern was the fold increase in serum peptide levels of these peptides that was equal or higher than 8 (fold change ≥ 8). In table 6, the fold increase of the serum peptide levels equal or higher than 8 (fold change ≥ 8) is marked with "***", confirming the diagnosis of ICH.

For the third serum peptide pattern (listed in Table 3 and 7) detected quantitative peptides had to show adjusted P value (adj. pval) ≤ 0.05 (adjusted p value ≤ 0.05 is marked with "**" in table 7). The second threshold for defining the second diagnostic peptide pattern was the fold increase in serum peptide levels of these peptides that was equal or higher than 10 (fold change ≥ 10). In table 7, the fold increase of the serum peptide levels equal or higher than 10 (fold change ≥ 10) is marked with "***", confirming the diagnosis of ICH.

For the fourth serum peptide pattern (listed in Table 4 and 8) detected quantitative peptides had to show adjusted P value (adj. pval) ≤ 0.05 (adjusted p value ≤ 0.05 is marked with "**" in table 8). The second threshold for defining the second diagnostic peptide pattern was the fold increase in serum peptide levels of these peptides that was equal or higher than 16 (fold change ≥ 16). In table 8, the fold increase of the serum peptide levels equal or higher than 16 (fold change ≥ 16) is marked with "***", confirming the diagnosis of ICH.

For each of four serum peptide patterns for diagnostics of ICH the fold-increase reflects the ratio of integrated peak areas in peptides and shows how many times the serum levels of the peptide are increased in ICH patients as compared with individuals without stroke and patients with AIS.

These four serum peptide patterns (Tables 1-8) are complexed sets of biomarkers for ICH diagnostics and differentiation from AIS. Their diagnostic value was checked during the approach for the invention, and their levels were increased in serum samples of ICH patients as determined with MS/MS (tandem mass spectrometry) signaling intensities. However, MS/MS (tandem mass spectrometry) and signaling intensity measurement are not the limitations of the invention. The peptides can be identified and measured (quantified) with different methods.

Hence, in table 5, 1-34 peptides: Glu-Phe-Val-Ser-Glu-Thr-Glu-Ser-Arg-Gly-Ser-Glu, Ala-Glu-Pro-Glu-Gly-Gly-Pro-Ala-Ser-Glu-Gly-Ala-Ala-Arg-Pro-Leu-Pro-Arg, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Phe-Val-Ser-Glu-Thr-Glu-Ser-Arg-Gly-Ser-Glu-Ser-Gly, Thr-Gln-Val-Ser-Thr-Gln-Ala-Glu-Gln-Leu-Arg, Lys-Phe-Asp-Lys-Ser-Lys-Leu, Glu-Leu-Gln-Gly-Val-Val-Pro-Arg, Thr-Ser-Ser-Ser-Ala-Ala-Pro-Gln-Arg-Pro-Ile, Ser-Val-Phe-Pro-Gln-Gln-Thr-Gly-Gln-Leu-Ala-Glu-Leu-Gln-Pro-Gln-Asp-Arg-Ala-Gly-Ala-Arg-Ala-Ser, Asn-Thr-Lys-Ser-Pro-Leu-Phe-Met-Gly-Lys-Val-Val-Asn-Pro-Thr-Gln-Lys, Tyr-Lys-His-Tyr-Asp-Gly-Ser-Tyr, Asp-Thr-Asn-Arg-Ala-Ser-Val-Gly-Gln-Asp-Ser-Pro-Glu-Pro-Arg, Phe-Ser-Pro-Val-Thr-Pro-Lys-Phe-Thr-Pro-Val-Ala-Ser, Asp-Ser-Gly-Glu-Gly-Asp-Phe-Leu-Ala-Glu-Gly-Gly-Gly-Val-Arg, Gln-Phe-Thr-Ser-Ser-Thr-Ser-Tyr-Asn-Arg-Gly-Asp-Ser, Arg-Ile-His-Trp-Glu-Ser-Ala-Ser, Val-Gly-Pro-Ala-Gly-Ala-Val-Gly-Pro-Arg, Asp-Arg-Asn-Leu-Pro-Ser-Asp-Ser-Gln-Asp-Leu-Gly-Gln-His-Gly, Lys-Lys-Pro-Leu-Thr-Ser-Ser-Ser-Ala-Ala-Pro-Gln-Arg-Pro-Ile-Ser-Thr-Gln, Asn-Asp-Asn-Glu-Glu-Gly-Phe-Phe, Ala-Leu-Thr-Ser-Glu-Glu-Ala-Glu-Arg-Ser-Asp-Gly-Asp-Pro-Val-Gln-Pro-Ala, His-Thr-Glu-Ala-Gln-Ile-Gln-Glu-Glu-Gly-Thr-Val, Ser-Thr-Ser-Tyr-Asn-Arg-Gly-Asp-Ser-Thr-Phe-Glu-Ser-Lys-Ser, Glu-Ile-Gln-Ile-Tyr-Lys-Lys-Leu-Arg, Lys-Glu-Ser-Ser-Ser-His-His-Pro-Gly-Ile-Ala-Glu-Phe-Pro-Ser, Thr-Pro-Leu-Gly-Gln-Gln-Gln-Pro-Ala-Pro-Arg, Tyr-Ser-Lys-Gln-Phe-Thr-Ser, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Ala-Leu-Gln-Leu-Asn-Asn-Arg-Gln-Ile, Val-Leu-Ser-Val-Leu-Lys-Gly-Glu-Glu, Ser-Leu-Val-Ile-Ser-Pro-Pro-Val-Arg-Thr-Ala-Thr-Val-Ser are quantitative peptides that were increased at least twice in ICH patients as compared with individuals without stroke and patients with AIS and the differences were statistically significant (adjusted p value ≤ 0.05 and fold change ≥ 2).

The next 16 peptides (from 35-50) in table 5:Tyr-Val-Ser-Asp-Ala-Phe-His-Lys-Ala-Phe, Gly-Ala-Asp-Gly-Val-Pro-Gly-Lys-Asp-Gly-Pro-Arg-Gly-Pro, Pro-Gly-Lys-Asp-Gly-Pro-Arg-Gly-Pro-Thr, Ser-Leu-Gly-Ser-Pro-Ser-Gly-Glu-Val, Ser-Asp-Leu-Gln-Ala-Gln-Ser-Lys-Gly-Asn-Pro-Glu-Gln-Thr-Pro-Val, Asp-Arg-Lys-Tyr-Glu-Glu-Val, Leu-Gln-Arg-Val-Lys-Arg-Leu-Pro-Leu-Glu-Tyr-Ser-Tyr, Ser-Glu-Gly-Ala-Ala-Arg-Pro-Leu-Pro-Arg, Ser-Lys-Gly-Ala-Gly-Gly-Gln-Gly-Lys-Val-Ala-Ser-Lys-Ile-Val-Gly-Pro-Ser-Gly, Glu-Glu-Glu-Leu-Val-Lys-Val-Arg-Glu-Lys-Gln-Leu-Ala, Glu-Glu-Gln-Leu-Glu-Glu-Glu-Glu-Ser-Ala-Arg-Gln, Ala-Gln-Ser-Gly-Pro-Val-Arg-Ser-Gly-Pro-Lys-Pro-Phe-Ser-Ala-Pro-Lys-Pro-Gln, Ala-Gln-Ser-Gly-Pro-Val-Arg-Ser-Gly-Pro-Lys-Pro-Phe-Ser-Ala-Pro-Lys-Pro-Gln-Thr, Ala-Leu-Glu-Asn-His-Gly-Phe, Gly-Gly-Thr-Gly-Gly-Ile-Gly-Gly-Val-Gly-Gly-Thr-Gly-Gly-Val-Gly-Asn-Arg-Ala-Pro-Arg, Val-Gly-Gly-Thr-Gly-Gly-Ile-Gly-Gly-Val-Gly-Gly-Thr-Gly-Gly-Val-Gly-Asn-Arg-Ala-Pro-Arg)are qualitative peptides detectable only in patients with ICH. If other peptides are detected or show ≥2-fold increase they do not correspond with intracranial hemorrhagic (ICH) stroke.

**Table 5. The serum peptide pattern 1 for diagnostics of ICH and differential diagnosis of ICH from AIS. List of ICH diagnostic peptides with amino acid sequence, adjusted p value, and fold change is given.**

| | Serial number | Source protein identifier* | Peptide sequence | Adjusted p value** | Fold change*** |
|---|---|---|---|---|---|
| Quantitative peptides | 1 | FIBA_HUMAN | Glu-Phe-Val-Ser-Glu- Thr-Glu-Ser-Arg-Gly-Ser- Glu | 0.000351 | 16.32921 |
| | 2 | CSF1_HUMAN | Ala-Glu-Pro-Glu-Gly-Gly- Pro-Ala-Ser-Glu-Gly-Ala- Ala-Arg-Pro-Leu-Pro-Arg | 0.003319 | 2.597295 |
| | 3 | ESAM_HUMAN | Thr-Thr-Asp-Gly-Ala-His- Pro-Gln-Pro-Ile | 0.003319 | 8.846694 |
| | 4 | TYB4_HUMAN | Thr-Glu-Thr-Gln-Glu- Lys-Asn-Pro-Leu-Pro- Ser-Lys-Glu-Thr | 0.003557 | 19.7557 |
| | 5 | TYB4_HUMAN | Lys-Thr-Glu-Thr-Gln- Glu-Lys-Asn-Pro-Leu- Pro-Ser-Lys-Glu-Thr | 0.003557 | 20.19883 |
| | 6 | FIBA_HUMAN | Phe-Val-Ser-Glu-Thr- Glu-Ser-Arg-Gly-Ser-Glu- Ser-Gly | 0.004475 | 2.217441 |
| | 7 | APOA4_HUMAN | Thr-Gln-Val-Ser-Thr-Gln- Ala-Glu-Gln-Leu-Arg | 0.005076 | 2.989061 |
| | 8 | TYB4Y_HUMAN | Lys-Phe-Asp-Lys-Ser- Lys-Leu | 0.005087 | 4.934849 |
| | 9 | F13A_HUMAN | Glu-Leu-Gln-Gly-Val-Val- Pro-Arg | 0.005161 | 4.178697 |
| | 10 | MARE1_HUMAN | Thr-Ser-Ser-Ser-Ala-Ala- Pro-Gln-Arg-Pro-Ile | 0.005264 | 7.437675 |
| | 11 | HEPC_HUMAN | Ser-Val-Phe-Pro-Gln- Gln-Thr-Gly-Gln-Leu- Ala-Glu-Leu-Gln-Pro- Gln-Asp-Arg-Ala-Gly- Ala-Arg-Ala-Ser | 0.005515 | 3.177922 |
| | 12 | A1AT_HUMAN | Asn-Thr-Lys-Ser-Pro- Leu-Phe-Met-Gly-Lys- Val-Val-Asn-Pro-Thr- Gln-Lys | 0.008191 | 4.415827 |
| | 13 | A2MG_HUMAN | Tyr-Lys-His-Tyr-Asp-Gly- Ser-Tyr | 0.00911 | 3.583354 |
| | 14 | PXDC2_HUMAN | Asp-Thr-Asn-Arg-Ala- Ser-Val-Gly-Gln-Asp-Ser- Pro-Glu-Pro-Arg | 0.010143 | 12.21539 |
| | 15 | ZYX_HUMAN | Phe-Ser-Pro-Val-Thr- Pro-Lys-Phe-Thr-Pro- Val-Ala-Ser | 0.010756 | 4.526763 |
| | 16 | FIBA_HUMAN | Asp-Ser-Gly-Glu-Gly- Asp-Phe-Leu-Ala-Glu- Gly-Gly-Gly-Val-Arg | 0.012001 | 2.493664 |
| | 17 | FIBA_HUMAN | Gln-Phe-Thr-Ser-Ser- Thr-Ser-Tyr-Asn-Arg- | 0.015284 | 3.731651 |
| | | | Gly-Asp-Ser | | |
| | 18 | CO3_HUMAN | Arg-Ile-His-Trp-Glu-Ser- Ala-Ser | 0.015442 | 28.58701 |
| | 19 | CO1A2_HUMAN | Val-Gly-Pro-Ala-Gly-Ala- Val-Gly-Pro-Arg | 0.015865 | 3.838523 |
| | 20 | SRGN_HUMAN | Asp-Arg-Asn-Leu-Pro- Ser-Asp-Ser-Gln-Asp- Leu-Gly-Gln-His-Gly | 0.017962 | 4.072656 |
| | 21 | MARE1_HUMAN | Lys-Lys-Pro-Leu-Thr-Ser- Ser-Ser-Ala-Ala-Pro-Gln- Arg-Pro-Ile-Ser-Thr-Gln | 0.020711 | 2.970456 |
| | 22 | FIBB_HUMAN | Asn-Asp-Asn-Glu-Glu- Gly-Phe-Phe | 0.023066 | 3.081567 |
| | 23 | CAVN2_HUMAN | Ala-Leu-Thr-Ser-Glu- Glu-Ala-Glu-Arg-Ser- Asp-Gly-Asp-Pro-Val- Gln-Pro-Ala | 0.023073 | 9.235255 |
| | 24 | A2MG_HUMAN | His-Thr-Glu-Ala-Gln-Ile- Gln-Glu-Glu-Gly-Thr-Val | 0.023119 | 4.267532 |
| | 25 | FIBA_HUMAN | Ser-Thr-Ser-Tyr-Asn- Arg-Gly-Asp-Ser-Thr- Phe-Glu-Ser-Lys-Ser | 0.025315 | 2.461149 |
| | 26 | APOB_HUMAN | Glu-Ile-Gln-Ile-Tyr-Lys- Lys-Leu-Arg | 0.025741 | 2.708401 |
| | 27 | FIBA_HUMAN | Lys-Glu-Ser-Ser-Ser-His- His-Pro-Gly-Ile-Ala-Glu- Phe-Pro-Ser | 0.025853 | 2.199607 |
| | 28 | LTBP2_HUMAN | Thr-Pro-Leu-Gly-Gln- | 0.026287 | 2.102014 |
| | | | Gln-Gln-Pro-Ala-Pro-Arg | | |
| | 29 | FIBA_HUMAN | Tyr-Ser-Lys-Gln-Phe- Thr-Ser | 0.030439 | 2.121467 |
| | 30 | TYB4_HUMAN | Lys-Asn-Pro-Leu-Pro- Ser-Lys-Glu-Thr | 0.030439 | 10.23718 |
| | 31 | FIBA_HUMAN | Ser-Tyr-Ser-Lys-Gln-Phe- Thr-Ser-Ser-Thr-Ser-Tyr- Asn-Arg-Gly | 0.037869 | 25.76081 |
| | 32 | CO4A_HUMAN | Ala-Leu-Gln-Leu-Asn- Asn-Arg-Gln-Ile | 0.038344 | 2.509442 |
| | 33 | APOC2_HUMAN | Val-Leu-Ser-Val-Leu-Lys- Gly-Glu-Glu | 0.040598 | 9.495219 |
| | 34 | BIN2_HUMAN | Ser-Leu-Val-Ile-Ser-Pro- Pro-Val-Arg-Thr-Ala-Thr- Val-Ser | 0.045049 | 2.072346 |
| Qualitative peptides | 35 | ANT3_HUMAN | Tyr-Val-Ser-Asp-Ala- Phe-His-Lys-Ala-Phe | ICH only | ICH only |
| | 36 | CO3A1_HUMAN | Gly-Ala-Asp-Gly-Val-Pro- Gly-Lys-Asp-Gly-Pro- Arg-Gly-Pro | ICH only | ICH only |
| | 37 | CO3A1_HUMAN | Pro-Gly-Lys-Asp-Gly- Pro-Arg-Gly-Pro-Thr | ICH only | ICH only |
| | 38 | FETUA_HUMAN | Ser-Leu-Gly-Ser-Pro-Ser- Gly-Glu-Val | ICH only | ICH only |
| | 39 | VTNC_HUMAN | Ser-Asp-Leu-Gln-Ala- Gln-Ser-Lys-Gly-Asn- Pro-Glu-Gln-Thr-Pro-Val | ICH only | ICH only |
| | 40 | TPM3_HUMAN | Asp-Arg-Lys-Tyr-Glu- | ICH only | ICH only |
| | | | Glu-Val | | |
| | 41 | CO8B_HUMAN | Leu-Gln-Arg-Val-Lys- Arg-Leu-Pro-Leu-Glu- Tyr-Ser-Tyr | ICH only | ICH only |
| | 42 | CSF1_HUMAN | Ser-Glu-Gly-Ala-Ala-Arg- Pro-Leu-Pro-Arg | ICH only | ICH only |
| | 43 | FLNA_HUMAN | Ser-Lys-Gly-Ala-Gly-Gly- Gln-Gly-Lys-Val-Ala-Ser- Lys-Ile-Val-Gly-Pro-Ser- Gly | ICH only | ICH only |
| | 44 | MYH9_HUMAN | Glu-Glu-Glu-Leu-Val- Lys-Val-Arg-Glu-Lys-Gln- Leu-Ala | ICH only | ICH only |
| | 45 | MYH9_HUMAN | Glu-Glu-Gln-Leu-Glu- Glu-Glu-Glu-Ser-Ala- Arg-Gln | ICH only | ICH only |
| | 46 | CAP1_HUMAN | Ala-Gln-Ser-Gly-Pro-Val- Arg-Ser-Gly-Pro-Lys-Pro- Phe-Ser-Ala-Pro-Lys- Pro-Gln | ICH only | ICH only |
| | 47 | CAP1_HUMAN | Ala-Gln-Ser-Gly-Pro-Val- Arg-Ser-Gly-Pro-Lys-Pro- Phe-Ser-Ala-Pro-Lys- Pro-Gln-Thr | ICH only | ICH only |
| | 48 | ITIH3 HUMAN | Ala-Leu-Glu-Asn-His- Gly-Phe | ICH only | ICH only |
| | 49 | MMRN1_HUMAN | Gly-Gly-Thr-Gly-Gly-Ile- Gly-Gly-Val-Gly-Gly-Thr- | ICH only | ICH only |
| | | | Gly-Gly-Val-Gly-Asn-Arg- Ala-Pro-Arg | | |
| | 50 | MMRN1_HUMAN | Val-Gly-Gly-Thr-Gly-Gly- Ile-Gly-Gly-Val-Gly-Gly- Thr-Gly-Gly-Val-Gly-Asn- Arg-Ala-Pro-Arg | ICH only | ICH only |

Hence, in table 6, the following 10 peptides: Glu-Phe-Val-Ser-Glu-Thr-Glu-Ser-Arg-Gly-Ser-Glu, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Ala-Leu-Thr-Ser-Glu-Glu-Ala-Glu-Arg-Ser-Asp-Gly-Asp-Pro-Val-Gln-Pro-Ala, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Val-Leu-Ser-Val-Leu-Lys-Gly-Glu-Glu are quantitative peptides that were increased at least 8 folds in ICH patients as compared with individuals without stroke and patients with AIS and the differences were statistically significant (adjusted p value ≤ 0.05 and fold change ≥ 8).

**Table 6. The serum peptide pattern 2 for diagnostics of ICH and differential diagnosis of ICH from AIS. List of ICH diagnostic peptides with amino acid sequence, adjusted p value, and fold change is given.**

| | Serial number | Source protein identifier* | Peptide sequence | Adjusted p value** | Fold change*** |
|---|---|---|---|---|---|
| Quantitative peptides | 1. | FIBA_HUMAN | Glu-Phe-Val-Ser-Glu-Thr- Glu-Ser-Arg-Gly-Ser-Glu | 0.000351 | 16.32921 |
| | 2. | ESAM_HUMAN | Thr-Thr-Asp-Gly-Ala-His- Pro-Gln-Pro-Ile | 0.003319 | 8.846694 |
| | 3. | TYB4_HUMAN | Thr-Glu-Thr-Gln-Glu-Lys- Asn-Pro-Leu-Pro-Ser-Lys- Glu-Thr | 0.003557 | 19.7557 |
| | 4. | TYB4_HUMAN | Lys-Thr-Glu-Thr-Gln-Glu- Lys-Asn-Pro-Leu-Pro-Ser- Lys-Glu-Thr | 0.003557 | 20.19883 |
| | 5. | PXDC2_HUMAN | Asp-Thr-Asn-Arg-Ala-Ser- Val-Gly-Gln-Asp-Ser-Pro- Glu-Pro-Arg | 0.010143 | 12.21539 |
| | 6. | CO3_HUMAN | Arg-Ile-His-Trp-Glu-Ser- Ala-Ser | 0.015442 | 28.58701 |
| | 7. | CAVN2_HUMAN | Ala-Leu-Thr-Ser-Glu-Glu- Ala-Glu-Arg-Ser-Asp-Gly- Asp-Pro-Val-Gln-Pro-Ala | 0.023073 | 9.235255 |
| | 8. | TYB4_HUMAN | Lys-Asn-Pro-Leu-Pro-Ser- Lys-Glu-Thr | 0.030439 | 10.23718 |
| | 9. | FIBA_HUMAN | Ser-Tyr-Ser-Lys-Gln-Phe- Thr-Ser-Ser-Thr-Ser-Tyr- Asn-Arg-Gly | 0.037869 | 25.76081 |
| | 10. | APOC2_HUMAN | Val-Leu-Ser-Val-Leu-Lys- Gly-Glu-Glu | 0.040598 | 9.495219 |

Hence, in table 7, the following 7 peptides: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Lys-Asn-Pro-Leu-Pro-Ser-Lys-Glu-Thr,Ser-Tyr-Ser-Lys-Gln-Phe-Thr-Ser-Ser-Thr-Ser-Tyr-Asn-Arg-Gly are quantitative peptides that were increased at least 10 folds in ICH patients as compared with individuals without stroke and patients with AIS and the differences were statistically significant (adjusted p value ≤ 0.05 and fold change ≥ 10).

**Table 7. The serum peptide pattern 3 for diagnostics of ICH and differential diagnosis of ICH from AIS. List of ICH diagnostic peptides with amino acid sequence, adjusted p value, and fold change is given.**

| | Serial number | Source protein identifier* | Peptide sequence | Adjusted p value** | Fold change*** |
|---|---|---|---|---|---|
| Quantitative peptides | 1. | FIBA_HUMAN | Glu-Phe-Val-Ser-Glu-Thr- Glu-Ser-Arg-Gly-Ser-Glu | 0.000351 | 16.32921 |
| | 2. | TYB4_HUMAN | Thr-Glu-Thr-Gln-Glu-Lys- Asn-Pro-Leu-Pro-Ser-Lys- Glu-Thr | 0.003557 | 19.7557 |
| | 3. | TYB4_HUMAN | Lys-Thr-Glu-Thr-Gln-Glu- Lys-Asn-Pro-Leu-Pro-Ser- Lys-Glu-Thr | 0.003557 | 20.19883 |
| | 4. | PXDC2_HUMAN | Asp-Thr-Asn-Arg-Ala-Ser- Val-Gly-Gln-Asp-Ser-Pro- Glu-Pro-Arg | 0.010143 | 12.21539 |
| | 5. | CO3_HUMAN | Arg-Ile-His-Trp-Glu-Ser- Ala-Ser | 0.015442 | 28.58701 |
| | 6. | TYB4_HUMAN | Lys-Asn-Pro-Leu-Pro-Ser- Lys-Glu-Thr | 0.030439 | 10.23718 |
| | 7. | FIBA_ HUMAN | Ser-Tyr-Ser-Lys-Gln-Phe- Thr-Ser-Ser-Thr-Ser-Tyr- Asn-Arg-Gly | 0.037869 | 25.76081 |

Hence, in table 8, the following 5 peptides: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Arg-Ile-His-Trp-Glu-Ser-Ala-Ser, Ser-Tyr-Ser-Lys-Gln-Phe-Thr-Ser-Ser-Thr-Ser-Tyr-Asn-Arg-Gly are quantitative peptides that were increased at least 16 folds in ICH patients as compared with individuals without stroke and patients with AIS and the differences were statistically significant (adjusted p value ≤ 0.05 and fold change ≥ 16).

**Table 8. The serum peptide pattern 4 for diagnostics of ICH and differential diagnosis of ICH from AIS. List of ICH diagnostic peptides with amino acid sequence, adjusted p value, and fold change is given.**

| | Serial number | Source protein identifier* | Peptide sequence | Adjusted p value** | Fold change*** |
|---|---|---|---|---|---|
| Quantitative peptides | 1. | FIBA_HUMAN | Glu-Phe-Val- Ser-Glu-Thr-Glu- Ser-Arg-Gly-Ser- Glu | 0.000351 | 16.32921 |
| | 2. | TYB4_HUMAN | Thr-Glu-Thr- Gln-Glu-Lys- Asn-Pro-Leu- Pro-Ser-Lys-Glu- Thr | 0.003557 | 19.7557 |
| | 3. | TYB4_HUMAN | Lys-Thr-Glu-Thr- Gln-Glu-Lys- Asn-Pro-Leu- Pro-Ser-Lys-Glu- Thr | 0.003557 | 20.19883 |
| | 4. | CO3_HUMAN | Arg-Ile-His-Trp- Glu-Ser-Ala-Ser | 0.015442 | 28.58701 |
| | 5. | FIBA_HUMAN | Ser-Tyr-Ser-Lys- Gln-Phe-Thr- Ser-Ser-Thr-Ser- Tyr-Asn-Arg-Gly | 0.037869 | 25.76081 |

### The advantages of invention are presented in the following tables

Table 5. The serum peptide pattern 1 for diagnostics of ICH and differential diagnosis of ICH from AIS. The peptides listed in Table 5 are qualitative and quantitative biomarkers of ICH since they distinguish patients with ICH from individuals without stroke and from patients with AIS. The sixteen qualitative markers (peptides 35-50 in table 5) are detectable only in patients with ICH, but not detectable in individuals without stroke and patients with AIS. Quantitative peptides (peptides 1-34 in table 5) are at least 2-folds increased in patients with ICH as compared with individuals without stroke and from patients with AIS. Their levels were determined with MS/MS (tandem mass spectrometry) signaling intensities. The upregulation of a particular peptide was determined by first integrating its peak area measured by a mass spectrometer across compared conditions here ICH and matched control groups (here individuals without stroke and patients with AIS). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words fold-increase shows how many times more the peptide is present in serum of the patients with ICH than in the individuals without stroke and patients with AIS. However, the method that we used (tandem mass spectrometry) for identification and quantification of the diagnostic peptides is not the limitation of the invention. Any other method for qualitative and quantitative peptide analysis can be used to identify and quantify these peptides.

In our analysis we have also considered the adjusted P value (adj. pval) which is the smallest significance level at which a particular comparison will be declared statistically significant as part of the multiple comparison such as ICH and respective control groups (individuals without stroke and patients with AIS). The threshold for selecting the peptides in the table 5 was as follow; adjusted P value (adj. pval) ≤ 0.05, fold-increase≥ 2 for the quantitative peptides. In the serum peptide pattern 1 for diagnostics of ICH and differential diagnosis of ICH from AIS the following 34 quantitative peptides: Glu-Phe-Val-Ser-Glu-Thr-Glu-Ser-Arg-Gly-Ser-Glu, Ala-Glu-Pro-Glu-Gly-Gly-Pro-Ala-Ser-Glu-Gly-Ala-Ala-Arg-Pro-Leu-Pro-Arg, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Phe-Val-Ser-Glu-Thr-Glu-Ser-Arg-Gly-Ser-Glu-Ser-Gly, Thr-Gln-Val-Ser-Thr-Gln-Ala-Glu-Gln-Leu-Arg, Lys-Phe-Asp-Lys-Ser-Lys-Leu, Glu-Leu-Gln-Gly-Val-Val-Pro-Arg, Thr-Ser-Ser-Ser-Ala-Ala-Pro-Gln-Arg-Pro-Ile, Ser-Val-Phe-Pro-Gln-Gln-Thr-Gly-Gln-Leu-Ala-Glu-Leu-Gln-Pro-Gln-Asp-Arg-Ala-Gly-Ala-Arg-Ala-Ser, Asn-Thr-Lys-Ser-Pro-Leu-Phe-Met-Gly-Lys-Val-Val-Asn-Pro-Thr-Gln-Lys, Tyr-Lys-His-Tyr-Asp-Gly-Ser-Tyr, Asp-Thr-Asn-Arg-Ala-Ser-Val-Gly-Gln-Asp-Ser-Pro-Glu-Pro-Arg, Phe-Ser-Pro-Val-Thr-Pro-Lys-Phe-Thr-Pro-Val-Ala-Ser, Asp-Ser-Gly-Glu-Gly-Asp-Phe-Leu-Ala-Glu-Gly-Gly-Gly-Val-Arg, Gln-Phe-Thr-Ser-Ser-Thr-Ser-Tyr-Asn-Arg-Gly-Asp-Ser, Arg-Ile-His-Trp-Glu-Ser-Ala-Ser, Val-Gly-Pro-Ala-Gly-Ala-Val-Gly-Pro-Arg, Asp-Arg-Asn-Leu-Pro-Ser-Asp-Ser-Gln-Asp-Leu-Gly-Gln-His-Gly, Lys-Lys-Pro-Leu-Thr-Ser-Ser-Ser-Ala-Ala-Pro-Gln-Arg-Pro-Ile-Ser-Thr-Gln, Asn-Asp-Asn-Glu-Glu-Gly-Phe-Phe, Ala-Leu-Thr-Ser-Glu-Glu-Ala-Glu-Arg-Ser-Asp-Gly-Asp-Pro-Val-Gln-Pro-Ala, His-Thr-Glu-Ala-Gln-Ile-Gln-Glu-Glu-Gly-Thr-Val, Ser-Thr-Ser-Tyr-Asn-Arg-Gly-Asp-Ser-Thr-Phe-Glu-Ser-Lys-Ser, Glu-Ile-Gln-Ile-Tyr-Lys-Lys-Leu-Arg, Lys-Glu-Ser-Ser-Ser-His-His-Pro-Gly-Ile-Ala-Glu-Phe-Pro-Ser, Thr-Pro-Leu-Gly-Gln-Gln-Gln-Pro-Ala-Pro-Arg, Tyr-Ser-Lys-Gln-Phe-Thr-Ser, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Ala-Leu-Gln-Leu-Asn-Asn-Arg-Gln-Ile, Val-Leu-Ser-Val-Leu-Lys-Gly-Glu-Glu, Ser-Leu-Val-Ile-Ser-Pro-Pro-Val-Arg-Thr-Ala-Thr-Val-Ser were quantified in serum samples from patients with ICH and in serum samples of the individuals without stroke and serum samples of patients with AIS and were at least 2 folds increased (≥ 2 folds) in serum samples of patients with ICH as compared with serum samples of individuals without stroke and patients with AIS .

Simultaneously the serum peptide pattern 1 comprises 16 qualitative peptides: Tyr-Val-Ser-Asp-Ala-Phe-His-Lys-Ala-Phe, Gly-Ala-Asp-Gly-Val-Pro-Gly-Lys-Asp-Gly-Pro-Arg-Gly-Pro, Pro-Gly-Lys-Asp-Gly-Pro-Arg-Gly-Pro-Thr, Ser-Leu-Gly-Ser-Pro-Ser-Gly-Glu-Val, Ser-Asp-Leu-Gln-Ala-Gln-Ser-Lys-Gly-Asn-Pro-Glu-Gln-Thr-Pro-Val, Asp-Arg-Lys-Tyr-Glu-Glu-Val, Leu-Gln-Arg-Val-Lys-Arg-Leu-Pro-Leu-Glu-Tyr-Ser-Tyr, Ser-Glu-Gly-Ala-Ala-Arg-Pro-Leu-Pro-Arg, Ser-Lys-Gly-Ala-Gly-Gly-Gln-Gly-Lys-Val-Ala-Ser-Lys-Ile-Val-Gly-Pro-Ser-Gly, Glu-Glu-Glu-Leu-Val-Lys-Val-Arg-Glu-Lys-Gln-Leu-Ala, Glu-Glu-Gln-Leu-Glu-Glu-Glu-Glu-Ser-Ala-Arg-Gln, Ala-Gln-Ser-Gly-Pro-Val-Arg-Ser-Gly-Pro-Lys-Pro-Phe-Ser-Ala-Pro-Lys-Pro-Gln, Ala-Gln-Ser-Gly-Pro-Val-Arg-Ser-Gly-Pro-Lys-Pro-Phe-Ser-Ala-Pro-Lys-Pro-Gln-Thr, Ala-Leu-Glu-Asn-His-Gly-Phe, Gly-Gly-Thr-Gly-Gly-Ile-Gly-Gly-Val-Gly-Gly-Thr-Gly-Gly-Val-Gly-Asn-Arg-Ala-Pro-Arg, Val-Gly-Gly-Thr-Gly-Gly-Ile-Gly-Gly-Val-Gly-Gly-Thr-Gly-Gly-Val-Gly-Asn-Arg-Ala-Pro-Arg that were detectable only in serum of the patients with ICH but were not detectable in serum samples of the individuals without stroke and serum samples of patients with AIS.

Table 6. The serum peptide pattern 2 for diagnostics of ICH and differential diagnosis of ICH from AIS. The peptides listed in Table 6 are quantitative biomarkers of ICH since they distinguish patients with ICH from individuals without stroke and from patients with AIS. The quantitative peptides listed in table 6 are at least 8-folds increased in patients with ICH as compared with individuals without stroke and from patients with AIS. Their levels were determined with MS/MS (tandem mass spectrometry) signaling intensities. The upregulation of a particular peptide was determined by first integrating its peak area measured by a mass spectrometer across compared conditions here ICH and matched control groups (here individuals without stroke and patients with AIS). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words, fold-increase shows how many times, more the peptide is present in serum of the patients with ICH than in the individuals without stroke and patients with AIS. However, the method that we used (tandem mass spectrometry) for quantification of the diagnostic peptides is not the limitation of the invention. Any other method for quantitative peptide analysis can be used to measure the increase in serum levels of these quantitative peptides in patients with ICH.

In our analysis we have also considered the adjusted P value (adj. pval) which is the smallest significance level at which a particular comparison will be declared statistically significant as part of the multiple comparison such as between patients with ICH and respective control groups (individuals without stroke and patients with AIS). The threshold for selecting the peptides in the table 6 was as follow; adjusted P value (adj. pval) ≤ 0.05, fold-increase≥ 8. In the serum peptide pattern 2 for diagnostics of ICH and differential diagnosis of ICH from AIS the following 10 peptides: Glu-Phe-Val-Ser-Glu-Thr-Glu-Ser-Arg-Gly-Ser-Glu, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Ala-Leu-Thr-Ser-Glu-Glu-Ala-Glu-Arg-Ser-Asp-Gly-Asp-Pro-Val-Gln-Pro-Ala, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Val-Leu-Ser-Val-Leu-Lys-Gly-Glu-Glu were quantified in serum samples from patients with ICH and in serum samples of the individuals without stroke and serum samples of patients with AIS and were at least 8 folds increased (≥ 8 folds) in serum samples of patients with ICH as compared with serum samples from individuals without stroke and patients with AIS .

Table 7. The serum peptide pattern 3 for diagnostics of ICH and differential diagnosis of ICH from AIS. The peptides listed in Table 7 are quantitative biomarkers of ICH since they distinguish patients with ICH from individuals without stroke and from patients with AIS. The quantitative peptides listed in table 7 are at least 10-folds increased in patients with ICH as compared with individuals without stroke and from patients with AIS. Their levels were determined with MS/MS (tandem mass spectrometry) signaling intensities. The upregulation of a particular peptide was determined by first integrating its peak area measured by a mass spectrometer across compared conditions here ICH and matched control groups (here individuals without stroke and patients with AIS). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words, fold-increase shows how many times more the peptide is present in serum of the patients with ICH than in the individuals without stroke and patients with AIS. However, the method that we used (tandem mass spectrometry) for quantification of the diagnostic peptides is not the limitation of the invention. Any other method for quantitative peptide analysis can be used to measure the increase in serum levels of these quantitative peptides in patients with ICH.

In our analysis we have also considered the adjusted P value (adj. pval) which is the smallest significance level at which a particular comparison will be declared statistically significant as part of the multiple comparison such as between patients with ICH and respective control groups (individuals without stroke and patients with AIS). The threshold for selecting the peptides in the table 7 was as follow; adjusted P value (adj. pval) ≤ 0.05, fold-increase≥ 10. In the serum peptide pattern 3 for diagnostics of ICH and differential diagnosis of ICH from AIS the following 7 peptides: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Lys-Asn-Pro-Leu-Pro-Ser-Lys-Glu-Thr,Ser-Tyr-Ser-Lys-Gln-Phe-Thr-Ser-Ser-Thr-Ser-Tyr-Asn-Arg-Gly were quantified in serum samples from patients with ICH and in serum samples of the individuals without stroke and serum samples of patients with AIS and were at least 10 folds increased (≥ 10 folds) in serum samples of patients with ICH as compared with serum samples from individuals without stroke and patients with AIS.

Table 8. The serum peptide pattern 4 for diagnostics of ICH and differential diagnosis of ICH from AIS. The peptides listed in Table 8 are quantitative biomarkers of ICH since they distinguish patients with ICH from individuals without stroke and from patients with AIS. The quantitative peptides listed in table 8 are at least 16-folds increased in patients with ICH as compared with individuals without stroke and from patients with AIS. Their levels were determined with MS/MS (tandem mass spectrometry) signaling intensities. The upregulation of a particular peptide was determined by first integrating its peak area measured by a mass spectrometer across compared conditions here ICH and matched control groups (here individuals without stroke and patients with AIS). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words fold-increase shows how many times more the peptide is present in serum of the patients with ICH than in the individuals without stroke and patients with AIS. However, the method that we used (tandem mass spectrometry) for quantification of the diagnostic peptides is not the limitation of the invention. Any other method for quantitative peptide analysis can be used to measure the increase in serum levels of these quantitative peptides in patients with ICH.

In our analysis we have also considered the adjusted P value (adj. pval) which is the smallest significance level at which a particular comparison will be declared statistically significant as part of the multiple comparison such as between patients with ICH and respective control groups (individuals without stroke and patients with AIS). The threshold for selecting the peptides in the table 8 was as follow; adjusted P value (adj. pval) ≤ 0.05, fold-increase≥ 16. In the serum peptide pattern 4 for diagnostics of ICH and differential diagnosis of ICH from AIS the following 5 peptides: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Arg-Ile-His-Trp-Glu-Ser-Ala-Ser, Ser-Tyr-Ser-Lys-Gln-Phe-Thr-Ser-Ser-Thr-Ser-Tyr-Asn-Arg-Gly were quantified in serum samples from patients with ICH and in serum samples of the individuals without stroke and serum samples of patients with AIS and were at least 16 folds increased (≥ 16 folds) in serum samples of patients with ICH as compared with serum samples from individuals without stroke and patients with AIS.

The following examples illustrate the present invention

### Example

### Solution and glassware quality

Prior to starting any steps of peptidomics method, all the solutions using LC-MS (Liquid Chromatography with mass spectrometry) grade water, solvents, reagents, and ultraclean glass and plasticware were prepared. Moreover, all solutions, buffers were prepared immediately before use and disposed of after one week of storage.

### Collection of serum samples

The blood samples were collected directly after the stroke patients have been admitted to the hospital by specialized medical staff. Additionally, as a control blood samples from individuals without stroke and from patients with AIS were collected. All research procedures were performed per the principles of the World Medical Association Declaration of Helsinki. Thus, the studied samples were sera from patients with ICH diagnosed routinely with imaging diagnostics methods computed tomography (CT) or magnetic resonance imaging (MRI) (n=5), individuals without stroke (n=5), and patients with AIS (n=5) diagnosed routinely with imaging diagnostics methods computed tomography (CT) or magnetic resonance imaging (MRI). The serum samples were obtained as follow: the clotted blood samples were centrifuged at 3000 × g for 10 min at 4 °C and then the serum was collected and stored at -80 °C immediately until analysis.

### Buffer preparation

First step was to prepare all the necessary buffers on the same day of peptidomics samples preparation.
1st buffer: citrate-phosphate buffer at pH 3.1 to 3.6 - (0.131 M citric acid/0.066 M Na2HPO4, NaCl 150 mM) and adjust the pH 3.1 to 3.6 with 1M NaOH prepared in LC-MS (Liquid Chromatography with mass spectrometry) grade water.
2^{nd} solution: 0.2% formic acid/methanol volume per volume (v/v) was prepared (final proportion of formic acid in methanol was 0.2%).
3^{rd} solution: 0.2% formic acid/water volume per volume (v/v) was prepared (final proportion of formic acid in water was 0.2%).
4^{th} wash buffer: water/5% methanol/0.2% formic acid volume per volume (v/v) was prepared (final proportion of methanol and formic acid in water was 5% and 0.2%, respectively).
5^{th} elution buffer: water/80% methanol/0.2% formic acid volume per volume (v/v) was prepared (final proportion of methanol and formic acid in water was 80% and 0.2%, respectively).

### Sample preparation

100 µL serum was taken from each sample and incubated with 2.9 ml of citrate-phosphate buffer- at pH 3.1-3.6 (with optimal results for pH 3.3) for 3-5 minutes (gentle and manual vertexing necessary to dissociate protein-peptide interactions) in the temperature below 4 to 8 °C e.g. on the ice. All the above mixtures were mixed e.g. centrifuged at 5000 ×g for 5 minutes at 4 °C. The supernatant was collected and diluted to 1:1 with 3 ml 0.2% FA in LC-MS (Liquid Chromatography with mass spectrometry) water volume per volume (v/v).

### One step purification of peptides+

For the purification of peptides, all the diluted samples (~6ml each) were subjected to the Oasis cartridges (hydrophilic-lipophilic balanced columns, 30 mg; Waters), and all the detailed steps are as follows.
i) Cartridge's condition: cartridge was conditioned with 0.2% formic acid/methanol volume per volume (v/v) (in this solution final proportion of formic acid in methanol was 0.2%), 1ml/cartridge.
ii) Equilibration of cartridge: the cartridge was equilibrated with 0.2% formic acid/water volume per volume (v/v) (in this solution final proportion of formic acid in water was 0.2%), 1ml/cartridge.
iii) Sample loading: 6 ml of a sample that this step was the supernatant collected after centrifugation was loaded on the cartridges (hydrophilic-lipophilic balanced columns).
iv) Washing steps: cartridge was washed three times with water containing 5% methanol/0.2% formic acid volume per volume (v/v) (in this solution final proportion of methanol and formic acid in water was 5% and 0.2%, respectively), volume for each wash was 1ml/cartridge.
v) Elution: the bound material (peptides) were eluted with 1ml water containing 80% methanol/0.2% formic acid volume per volume (v/v) (in this solution final proportion of methanol and formic acid in water was 80% and 0.2%, respectively) and then diluted to water/40% methanol/0.2% formic acid volume per volume (v/v) (in this solution final proportion of methanol and formic acid in water was 40% and 0.2%, respectively).

### Peptides preparation for mass spectrometry analysis

The serum peptides sample obtained after peptides purification were spun through 3 kilo Dalton/kDa molecular weight cutoff (MWCO) ultrafiltration devices (AmiconUltra2Centrifugal Filters, Ultracel-3k, 2 mL: Millipore). Briefly, Amicon-Ultracel-3 kilo Dalton/kDa centrifugal filter was rinsed before use (the 3kDa ultrafiltration device was rinsed with 40% methanol with LC-MS (Liquid Chromatography with mass spectrometry) grade water volume per volume (v/v), 1ml water/column, and centrifuged at 4000 x g, 30 min, 4 °C). Next, 3kDa ultrafiltration device was inserted into the new collecting tube. Lastly, eluted and diluted peptide solution (~2ml) was pipetted into the 3kDa ultrafiltration device, taking care not to touch the membrane with the pipette tip. Then the 3kDa ultrafiltration device was span at 4000 x g, 120-135 min, 4 °C. The filtrate (peptides) was collected and then lyophilized to dryness. All dried peptide samples were stored at -80 until the mass spectrometry analysis.

### Sequencing of peptides with tandem mass spectrometry

The serum peptides obtained from sera of ICH patients and individuals without stroke and patients with AIS were further subjected to the LC-MS/MS (Liquid Chromatography with tandem mass spectrometry) analysis. The eluted and dried serum peptide samples were resuspended in 30 µl of loading buffer composed of 0.08% trifluoroacetic acid (TFA) in water with 2.5% acetonitrile (ACN). Indexed retention time (iRT) peptides (Biognosys) were added according to the manufacturer's guidelines. Then 6 µl of the dissolved sample were injected into UltiMate^{™} 3000 RSLCnano liquid chromatograph (Thermo Scientific) online coupled with Orbitrap Exploris 480 mass spectrometer (MS) (Thermo Scientific). µ-precolumn C18 trap cartridges (300 µm i.d.) and 5 mm length packed with C18 PepMap100 sorbent with PepMap 5 µm sorbent (P/N: 160454, Thermo Fisher Scientific) were used to concentrate and desalt serum peptides using a 5 µl/min flow of Loading buffer. Peptides were then eluted on 75 µm ID and 150 mm length fused-silica analytical column packed with PepMap 2 µm sorbent (P/N: 164534, Thermo Scientific). Analytical peptide separation was performed by a non-linear increase of a mobile phase B (0.1% formic acid (FA) in ACN) in a mobile phase A (0.1% FA in water). A non-linear gradient started at 2.5% B linearly increasing up to 35% B in 80 min, followed by a linear increase up to 60% B in the next 15 min with a flow rate of 300 nl/min. Serum peptides eluting from the column were ionized in a nano-electrospray ion source (NSI) and were introduced to Exploris 480 (Thermo Scientific).

### Mass spectrometry measurements for qualitative analysis

DDA(data-dependent acquisition) method has been used for qualitative peptidomics analysis. The data-dependent acquisition method was prepared in our laboratory. Briefly, Exploris 480 acquired the data in data-dependent peptide mode. The full scan was operated in profile mode with 120000 resolution, scanning the precursor range from m/z 350 Th to m/z 1650 Th. Normalized AGC (Automatic Gain Control) target was set to 300% with 100 msec maximum injection time. Each full scan was followed by fragmentation of the top 20 the most intense precursor ions and acquisition if their MS/MS spectra. The dynamic mass exclusion was set to 20 sec after the first precursor ion fragmentation. Precursor isotopologues were excluded, and mass tolerance was set to 10 ppm. Minimum precursor ion intensity was set to 3.0e3, and only precursor charge states of +1 to +6 were included in the experiment. The precursor isolation window was set to 1.6 Th. Normalized collision energy type with fixed collision energy mode was selected. The collision energy was set to 30%. Orbitrap resolution was set to 60000. Normalized AGC (Automatic Gain Control) target was set to 100% with an automatic setting of maximum injection time, and data type was centroid.

### Mass spectrometry measurements for quantitative analysis

DIA (data-independent acquisition) method has been used for quantitative peptidomics analysis. LC (Liquid Chromatography) separation parameters were kept identical to DDA (data-dependent acquisition) acquisition. Orbitrap Exploris 480 mass spectrometer operated in positive polarity data-independent mode (DIA) accompanied by a full-scan in profile mode with 60000 resolution. The full-scan range was set from m/z 350 Th up to m/z 1450 Th, and the normalized AGC (Automatic Gain Control) target was set to 300% with 100 msec maximum injection time. Each DIA (data-independent acquisition) cycle was accompanied by the acquisition of 62 precursor windows/scan events. DIA (data-independent acquisition) precursor range was set from m/z 350 Th up to m/z 1100 Th with 12 Th window width and 1 Th window overlap. Normalized collision energy type with fixed collision energy mode was selected to fragment precursors included within each isolation window. The collision energy was set to 30% and Orbitrap resolution in DIA (data-independent acquisition) mode was set to 30000. Normalized AGC (Automatic Gain Control) target was set to 1000% with automatic setting of maximum injection time while data type was profile.

### Data analysis for qualitative peptidomics

First, multi-search engine strategy has been applied for comprehensive qualitative analysis of serum peptidome. DDA (data-dependent acquisition) data were centroided and converted to mzML and mzXML format using MSconvert (version: 3.0.19094). Converted MS data were searched using MSFragger 3.4 embedded in Fragpipe (v.15) suite and Comet (Release 2020.01, revision 2) embedded in TPP 5.2.0 against *Homo sapiens* SwissProt+TrEMBL reference database concatenated with indexed retention time (iRT) peptide sequence (Biognosys), decoy reverse target sequences and contaminants. The search database was constructed in Fragpipe (v.15) suite. The following search settings were used for MSFragger: precursor mass tolerance was set to +-8 ppm and fragment mass tolerance to 10 ppm. Enzyme digestion was set to non-specific and peptide length was set from 7 to 45 amino acids. Peptide mass range was set from 200 to 5000 Da. Variable modifications were set to: methionine oxidation, protein N-term acetylation, and cysteinylation of cysteine. Data were mass recalibrated, and automatic parameter optimization setting was used to tune fragment mass tolerance. Output file format was set to pep.XML. The following search settings were used for Comet: precursor mass tolerance was set to 8 ppm and fragment mass tolerance to 8 ppm. The rest of the settings were identical to MSFragger. The search results (pep.XML files) were processed with PeptideProphet and iProphet as part of the Trans-Proteomic Pipeline (TPP) 5.2.0. Resulting recalculated pep.XML files with peptide iprobabilities (iPROB values) were further processed in Skyline-daily (64-bit, 20.1.9.234).

### Data analysis for quantitative peptidomics

A spectral library was built from recalculated pep.XML files in Skyline-daily (64-bit, 20.1.9.234). In "Peptide settings" a "Library" tab was selected. An option of "Build" was selected and in the newly popped-up window the "Cut-off score" was set to 0.99 (considers only peptides with false discovery rate (FDR) < 0.01 or 0.99 < iPROB value) and as an indexed retention time standard peptides were selected "Biognosys-11 (iRT-C18)". All recalculated pep.XML files were loaded and a process of building-up the spectral library was initiated. Next, in the "Digestion" tab in "Background proteome" section the "add" option was selected. In the newly popped up window the background proteome was created from the .FASTA file that was previously used as a reference search library (SwissProt+TrEMBL reference protein database concatenated with indexed retention time peptide sequence (Biognosys), decoy reverse target sequences and contaminants). Next, in the "Library" tab, "Libraries" section the newly built spectral library was selected and the "Explore" button was clicked to explore the library. In the library window an "Associate proteins" option was checked and the "Add all" button was clicked. All peptides including non-unique were added to the document. Skyline automatically generated a retention time calculator based on the indexed retention time peptide retention time values observed in recalculated pep.XML files. The Skyline file was saved on the PC hard disc drive along with newly built spectral library (.blib format).

### Data extraction for quantitative peptidomics

The Skyline file saved in the previous step was continued to set the extraction settings properly. Peptide peak areas reflecting the peptide quantity in sample (quantitative values) were extracted from DIA (data-independent acquisition) data in Skyline-daily (64-bit, 20.1.9.234) based on the peptide-product ion pairs (transitions) listed in the spectral library generated in the previous step. In the "Peptide settings" tab, the "Max. missed cleavages" was set to 0. Next, in the "Filter" sub-tab a maximum peptide length was set from 4 to 200 amino acids. The "Exclude N terminal AAs" option was set to zero. A function of the "Auto-select all matching peptides" was selected. In the "Modifications" sub-tab, no modifications were selected. In the "Library" sub-tab, in the "Libraries" window, a spectral library that was created in the previous step was selected. Other settings in the tab were left default. In "Transition settings" tab, the "Prediction" sub-tab was left default. In the "Filter" sub-tab +1, +2, +3, +5, +6 peptide precursor charges were specified. Ion charges were set to +1 and +2. Ion type was set to y and b. Product ion selection was set as follows: in the "From:" window the "ion 4" and in the "To:" window the "last ion" options were selected. The "Auto select all matching transition" option was selected at the bottom of the sub-tab and in the "Special ions:" menu the "N-terminal to Proline" option was selected. In the "Library" sub-tab, the "Ion match tolerance" window was set to 0.05 m/z and only peptides that have at least 4 product ions were kept in analysis. In addition, if more product ions were available per peptide, then the 6 most intense were picked-up from the filtered product ions. A function of the "From filtered ion charges and types" was chosen. In the "Instrument" sub-tab we included product ions from m/z 350 up to m/z 1100. The "Method match tolerance m/z" window was set to 0.055 m/z mass tolerance. MS1 filtering was set to "none" in the full scan sub-tab and in MS/MS filtering sub-section, the "Acquisition method" window the "DIA" option was selected and in the "Product mass analyzer" the "Orbitrap" option was selected. In the "Isolation scheme:" the "Add" option was selected and in the "Edit isolation scheme" pop-up window an option of the "Prespecified isolation windows" was selected. The "Import" isolation windows from DIA (data-independent acquisition).raw file option was then selected, and the isolation scheme was read from a DIA (data-independent acquisition) raw file. Back in the "Full-Scan" sub-tab in the "Resolving power:" section the resolving power was set to 30000 at 200 m/z. In the retention time filtering sub-section "Use only scans within 5 minutes of MS/MS IDs" was specified. Under the "Refine" sub-tab, the "Document" section of the "Advanced" window the "Min transitions per precursor" option was set to 4. Empty proteins were removed from the document. An equal number of reverse sequence decoys via the "Add Decoys" function in the "Refine" sub-tab was added. In the "Add Decoy Peptides" pop-up window a reverse sequence decoy generation method to create decoy peptides was selected. Following, DIA (data-independent acquisition) ".raw files" were imported. mProphet model to reintegrate peptide product ion peak boundaries in product ion chromatograms was trained as follows: in the "Refine" sub-tab the "Reintegrate" function was selected. In the "Reintegrate" pop-up window in the "Peak scoring model:" the "Add" option was selected. Following, in the "Edit Peak Scoring Model" mProphet model was activated. mProphet model was trained using targets and decoys. The "Score rows" with negative "Weight" and/or "Percentage Contribution" (red highlighted) were removed, and the mProphet model was then re-trained. New mProphet peak scoring model was then selected in the "Edit peak scoring Model" window and applied for reintegration of new peak boundaries. "Export report" function to export a summary of all dependencies required for MSstats 4.0.1. was used to generate quantitative report for downstream analysis.

### Statistical data analysis for quantitative peptidomics

DIA (data-independent acquisition) statistical data analysis performed in MSstats R-module. Statistical analysis of Skyline extracted quantitative DIA (data-independent acquisition) data was performed in R (version 4.0.0) package MSstats 4.0.1. The protein column was combined with the peptide sequence column to preserve analysis at peptide level, this formatting prevents summing peptide intensities originating from a single protein in MSstats. Extracted peakgroups were reduced by filtering on mProphet q-value < 0.01 cut-off. "SkylinetoMSstatsFormat" function was set to keep proteins with one feature and to transform Skyline output to MSstats compatible input. Further, peptide intensities were log2 transformed and quantile normalized. Differential serum peptide quantitation across intracranial hemorrhagic (ICH) stroke and individuals without stroke and patients with AIS were performed pairwise via mixed-effect models implemented in MSstats "groupComparison" function. p-values were adjusted using the Benjamini-Hochberg method and output result matrix was exported for downstream analyses.

### Serum peptide patterns for diagnostics of intracranial hemorrhagic stroke in humans and differential diagnosis of intracranial hemorrhagic stroke from acute ischemic stroke

The invention reveals the accurate, multi-biomarker peptides with the biological mechanism for intracranial hemorrhagic (ICH) stroke. In precisely, ICH is intracranial hemorrhage, which is the blood flooding in the side of the brain. This kind of stroke is severe because it generates many wounds inside the brain and blood vessels. Due to the wound in the brain, blood vessels, cells, tissue of the patients, their body naturally tries to recover it by inducing the many wound healing mechanisms. Therefore, the first and natural mechanism is to activate the blood clotting mechanism to control the blood flows from the wound regions. This step of the blood clotting mechanism gathers the collagenase at the wound site. Collagenase forms the first transparent cover on the wound region. This collagenase layer acts as the base of further platelet molecules to bind on the wound site. Then one of the major components of the wound healing process is the binding of fibrinogen and giving the entire clotting complex strength. Lastly, the enzyme fibrin acts on this complex, makes a uniform layer, and completes the healing mechanism. Hence, it has the possibility to find peptide biomarkers from each incidence happening during ICH stroke developments. In the inclusion, our peptidomics approach reveals the peptides are identified and quantified as the response of intracranial hemorrhagic (ICH) stroke having clinical conditions corresponding to wound healing, and blood clotting mechanism. In the following list of biomarkers peptide candidates indicate developing point-of-care diagnostic (POCD) kits.

The invention provides four serum peptide patterns for diagnostics of ICH and differential diagnosis of ICH from AIS. These peptide patters are lists of unique serum biomarker peptides specific to ICH. Selection of up-regulated peptides in intracranial hemorrhagic (ICH) stroke compared to respective control groups (individuals without stroke and patients with AIS) (Table 5-8). Peptides are biomarkers of ICH since they distinguish it from individuals without stroke and patients with AIS groups based on their increased level determined from corresponding MS/MS (tandem mass spectrometry) signaling intensities. The upregulation of a particular peptide was determined by first integrating its peak area measured by a mass spectrometer across compared conditions here ICH and matched control groups (here individuals without stroke and patients with AIS). The integrated peak area reflects the number of peptide molecules in the measured sample. Next, by relative comparison, of integrated peak areas across the compared groups the fold-increase increase is calculated. Fold-increase reflects the ratio of integrated peak areas in compared conditions and is proportional to the ratio of number of the peptide molecules in compared conditions. In other words fold-increase shows how many times more the peptide is present in serum of the patients with ICH than in the individuals without stroke and patients with AIS. However, the method that we used (tandem mass spectrometry) for identification and quantification of the diagnostic peptides is not the limitation of the invention. Any other method for qualitative and quantitative peptide analysis can be used to identify and quantify these peptides. In our analysis we have also considered the adjusted P value (adj. pval) which is the smallest significance level at which a particular comparison will be declared statistically significant as part of the multiple comparison such as ICH and respective control groups (individuals without stroke and patients with AIS). The threshold for selecting the serum peptide pattern 1 (table 5) was as follow; adjusted P value (adj. pval) ≤ 0.05, and fold-increase≥ 2 for quantitative peptides. The serum peptide pattern 1 comprises of the following 34 quantitative peptides: Glu-Phe-Val-Ser-Glu-Thr-Glu-Ser-Arg-Gly-Ser-Glu, Ala-Glu-Pro-Glu-Gly-Gly-Pro-Ala-Ser-Glu-Gly-Ala-Ala-Arg-Pro-Leu-Pro-Arg, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Phe-Val-Ser-Glu-Thr-Glu-Ser-Arg-Gly-Ser-Glu-Ser-Gly, Thr-Gln-Val-Ser-Thr-Gln-Ala-Glu-Gln-Leu-Arg, Lys-Phe-Asp-Lys-Ser-Lys-Leu, Glu-Leu-Gln-Gly-Val-Val-Pro-Arg, Thr-Ser-Ser-Ser-Ala-Ala-Pro-Gln-Arg-Pro-Ile, Ser-Val-Phe-Pro-Gln-Gln-Thr-Gly-Gln-Leu-Ala-Glu-Leu-Gln-Pro-Gln-Asp-Arg-Ala-Gly-Ala-Arg-Ala-Ser, Asn-Thr-Lys-Ser-Pro-Leu-Phe-Met-Gly-Lys-Val-Val-Asn-Pro-Thr-Gln-Lys, Tyr-Lys-His-Tyr-Asp-Gly-Ser-Tyr, Asp-Thr-Asn-Arg-Ala-Ser-Val-Gly-Gln-Asp-Ser-Pro-Glu-Pro-Arg, Phe-Ser-Pro-Val-Thr-Pro-Lys-Phe-Thr-Pro-Val-Ala-Ser, Asp-Ser-Gly-Glu-Gly-Asp-Phe-Leu-Ala-Glu-Gly-Gly-Gly-Val-Arg, Gln-Phe-Thr-Ser-Ser-Thr-Ser-Tyr-Asn-Arg-Gly-Asp-Ser, Arg-Ile-His-Trp-Glu-Ser-Ala-Ser, Val-Gly-Pro-Ala-Gly-Ala-Val-Gly-Pro-Arg, Asp-Arg-Asn-Leu-Pro-Ser-Asp-Ser-Gln-Asp-Leu-Gly-Gln-His-Gly, Lys-Lys-Pro-Leu-Thr-Ser-Ser-Ser-Ala-Ala-Pro-Gln-Arg-Pro-Ile-Ser-Thr-Gln, Asn-Asp-Asn-Glu-Glu-Gly-Phe-Phe, Ala-Leu-Thr-Ser-Glu-Glu-Ala-Glu-Arg-Ser-Asp-Gly-Asp-Pro-Val-Gln-Pro-Ala, His-Thr-Glu-Ala-Gln-Ile-Gln-Glu-Glu-Gly-Thr-Val, Ser-Thr-Ser-Tyr-Asn-Arg-Gly-Asp-Ser-Thr-Phe-Glu-Ser-Lys-Ser, Glu-Ile-Gln-Ile-Tyr-Lys-Lys-Leu-Arg, Lys-Glu-Ser-Ser-Ser-His-His-Pro-Gly-Ile-Ala-Glu-Phe-Pro-Ser, Thr-Pro-Leu-Gly-Gln-Gln-Gln-Pro-Ala-Pro-Arg, Tyr-Ser-Lys-Gln-Phe-Thr-Ser, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Ala-Leu-Gln-Leu-Asn-Asn-Arg-Gln-Ile, Val-Leu-Ser-Val-Leu-Lys-Gly-Glu-Glu, Ser-Leu-Val-Ile-Ser-Pro-Pro-Val-Arg-Thr-Ala-Thr-Val-Ser that were quantified in serum samples from patients with ICH and in serum samples of the individuals without stroke and serum samples of patients with AIS and were at least 2 folds increased (≥ 2 folds) in serum samples of patients with ICH as compared with serum samples of individuals without stroke and patients with AIS (Table 5). Simultaneously the serum peptide patter 1 comprises 16 qualitative peptides:Tyr-Val-Ser-Asp-Ala-Phe-His-Lys-Ala-Phe, Gly-Ala-Asp-Gly-Val-Pro-Gly-Lys-Asp-Gly-Pro-Arg-Gly-Pro, Pro-Gly-Lys-Asp-Gly-Pro-Arg-Gly-Pro-Thr, Ser-Leu-Gly-Ser-Pro-Ser-Gly-Glu-Val, Ser-Asp-Leu-Gln-Ala-Gln-Ser-Lys-Gly-Asn-Pro-Glu-Gln-Thr-Pro-Val, Asp-Arg-Lys-Tyr-Glu-Glu-Val, Leu-Gln-Arg-Val-Lys-Arg-Leu-Pro-Leu-Glu-Tyr-Ser-Tyr, Ser-Glu-Gly-Ala-Ala-Arg-Pro-Leu-Pro-Arg, Ser-Lys-Gly-Ala-Gly-Gly-Gln-Gly-Lys-Val-Ala-Ser-Lys-Ile-Val-Gly-Pro-Ser-Gly, Glu-Glu-Glu-Leu-Val-Lys-Val-Arg-Glu-Lys-Gln-Leu-Ala, Glu-Glu-Gln-Leu-Glu-Glu-Glu-Glu-Ser-Ala-Arg-Gln, Ala-Gln-Ser-Gly-Pro-Val-Arg-Ser-Gly-Pro-Lys-Pro-Phe-Ser-Ala-Pro-Lys-Pro-Gln, Ala-Gln-Ser-Gly-Pro-Val-Arg-Ser-Gly-Pro-Lys-Pro-Phe-Ser-Ala-Pro-Lys-Pro-Gln-Thr, Ala-Leu-Glu-Asn-His-Gly-Phe, Gly-Gly-Thr-Gly-Gly-Ile-Gly-Gly-Val-Gly-Gly-Thr-Gly-Gly-Val-Gly-Asn-Arg-Ala-Pro-Arg, Val-Gly-Gly-Thr-Gly-Gly-Ile-Gly-Gly-Val-Gly-Gly-Thr-Gly-Gly-Val-Gly-Asn-Arg-Ala-Pro-Arg that were detectable only in serum of the patients with ICH but were not detectable in serum samples of the individuals without stroke and serum samples of patients with AIS (Table 5).

As grade of the fold increase of the 34 quantitative peptides corresponds with their predictive significance for ICH diagnostics, we also defined the serum peptide pattern 2 (a panel of 10 peptides), the serum peptide pattern 3 (a panel of 7 peptides) and the serum peptide pattern 4 (a panel of 5 peptides) which can be used alone for ICH diagnostics or differentiation of ICH from AIS when their fold increase in serum of patients with ICH is equal or higher than 8, 10 and 16 folds, respectively as compared with serum levels of these peptides in patients without stroke and in patients with AIS.

The serum peptide pattern 2 for diagnostics of ICH and differential diagnosis of ICH from AIS is a set of quantitative peptides listed in Table 6. These quantitative peptides are at least 8-folds increased in patients with ICH as compared with individuals without stroke and from patients with AIS. Their levels were determined with MS/MS (tandem mass spectrometry) signaling intensities. The upregulation of a particular peptide was determined by first integrating its peak area measured by a mass spectrometer across compared conditions here ICH and matched control groups (here individuals without stroke and patients with AIS). The integrated peak area reflects the number of peptide molecules in the measured sample. Next, by relative comparison, of integrated peak areas across the compared groups the fold-increase increase was calculated. Fold-increase reflects the ratio of integrated peak areas in compared conditions and is proportional to the ratio of number of the peptide molecules in compared conditions. In other words fold-increase shows how many times more the peptide is present in serum of the patients with ICH than in the individuals without stroke and patients with AIS. However, the method that we used (tandem mass spectrometry) for quantification of the diagnostic peptides is not the limitation of the invention. Any other method for quantitative peptide analysis can be used to measure the increase in serum levels of these quantitative peptides in patients with ICH.

In our analysis we have also considered the adjusted P value (adj. pval) which is the smallest significance level at which a particular comparison will be declared statistically significant as part of the multiple comparison such as between patients with ICH and respective control groups (individuals without stroke and patients with AIS). The threshold for selecting the peptide pattern 2 ( Table 6) was as follow; adjusted P value (adj. pval) ≤ 0.05, fold-increase≥ 8. The serum peptide pattern 2 for diagnostics of ICH and differential diagnosis of ICH from AIS is defined by the following 10 peptides: Glu-Phe-Val-Ser-Glu-Thr-Glu-Ser-Arg-Gly-Ser-Glu, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Ala-Leu-Thr-Ser-Glu-Glu-Ala-Glu-Arg-Ser-Asp-Gly-Asp-Pro-Val-Gln-Pro-Ala, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Val-Leu-Ser-Val-Leu-Lys-Gly-Glu-Glu that are at least 8 folds increased (≥ 8 folds) in serum samples of patients with ICH as compared with serum samples from individuals without stroke and patients with AIS.

The serum peptide pattern 3 for diagnostics of ICH and differential diagnosis of ICH from AIS is a set of quantitative peptides listed in Table 7. These quantitative peptides are at least 10-folds increased in patients with ICH as compared with individuals without stroke and from patients with AIS. Their levels were determined with MS/MS (tandem mass spectrometry) signaling intensities. The upregulation of a particular peptide was determined by first integrating its peak area measured by a mass spectrometer across compared conditions here ICH and matched control groups (here individuals without stroke and patients with AIS). The integrated peak area reflects the number of peptide molecules in the measured sample. Next, by relative comparison, of integrated peak areas across the compared groups the fold-increase increase was calculated. Fold-increase reflects the ratio of integrated peak areas in compared conditions and is proportional to the ratio of number of the peptide molecules in compared conditions. In other words, fold-increase shows how many times more the peptide is present in serum of the patients with ICH than in the individuals without stroke and patients with AIS. However, the method that we used (tandem mass spectrometry) for quantification of the diagnostic peptides is not the limitation of the invention. Any other method for quantitative peptide analysis can be used to measure the increase in serum levels of these quantitative peptides in patients with ICH.

In our analysis we have also considered the adjusted P value (adj. pval) which is the smallest significance level at which a particular comparison will be declared statistically significant as part of the multiple comparison such as between patients with ICH and respective control groups (individuals without stroke and patients with AIS). The threshold for selecting the peptide pattern 3 ( Table 7) was as follow; adjusted P value (adj. pval) ≤ 0.05, fold-increase≥ 10. The serum peptide pattern 3 for diagnostics of ICH and differential diagnosis of ICH from AIS is defined by the following 7 peptides: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Lys-Asn-Pro-Leu-Pro-Ser-Lys-Glu-Thr,Ser-Tyr-Ser-Lys-Gln-Phe-Thr-Ser-Ser-Thr-Ser-Tyr-Asn-Arg-Gly that are at least 10 folds increased (≥ 10 folds) in serum samples of patients with ICH as compared with serum samples from individuals without stroke and patients with AIS.

The serum peptide pattern 4 for diagnostics of ICH and differential diagnosis of ICH from AIS is a set of quantitative peptides listed in Table 8. These quantitative peptides are at least 16-folds increased in patients with ICH as compared with individuals without stroke and from patients with AIS. Their levels were determined with MS/MS (tandem mass spectrometry) signaling intensities. The upregulation of a particular peptide was determined by first integrating its peak area measured by a mass spectrometer across compared conditions here ICH and matched control groups (here individuals without stroke and patients with AIS). The integrated peak area reflects the number of peptide molecules in the measured sample. Next, by relative comparison, of integrated peak areas across the compared groups the fold-increase increase was calculated. Fold-increase reflects the ratio of integrated peak areas in compared conditions and is proportional to the ratio of number of the peptide molecules in compared conditions. In other words, fold-increase shows how many times more the peptide is present in serum of the patients with ICH than in the individuals without stroke and patients with AIS. However, the method that we used (tandem mass spectrometry) for quantification of the diagnostic peptides is not the limitation of the invention. Any other method for quantitative peptide analysis can be used to measure the increase in serum levels of these quantitative peptides in patients with ICH.

In our analysis we have also considered the adjusted P value (adj. pval) which is the smallest significance level at which a particular comparison will be declared statistically significant as part of the multiple comparison such as between patients with ICH and respective control groups (individuals without stroke and patients with AIS). The threshold for selecting the peptide pattern 4 ( Table 8) was as follow; adjusted P value (adj. pval) ≤ 0.05, fold-increase≥ 16. The serum peptide pattern 4 for diagnostics of ICH and differential diagnosis of ICH from AIS is defined by the following 5 peptides: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Arg-Ile-His-Trp-Glu-Ser-Ala-Ser, Ser-Tyr-Ser-Lys-Gln-Phe-Thr-Ser-Ser-Thr-Ser-Tyr-Asn-Arg-Gly that are at least 16 folds increased (≥ 16 folds) in serum samples of patients with ICH as compared with serum samples from individuals without stroke and patients with AIS.

The invention provides 4 serum peptide patterns (Tables 5-8) that are sets of unique biomarker serum peptides specific to ICH. As a result, it will enhance confidence of accurate diagnosis because considering a single biomarker for stroke might be too risky to rely on an accurate diagnosis. In addition, our peptidomics analysis indicates that the unique and potential peptide multi-biomarkers ICH are derived from several proteins such as Fibrinogen alpha chain [Cleaved into: Fibrinopeptide A; Fibrinogen alpha chain] (FGA) (FIBA_HUMAN) and these peptides are:

Glu-Phe-Val-Ser-Glu-Thr-Glu-Ser-Arg-Gly-Ser-Glu
Phe-Val-Ser-Glu-Thr-Glu-Ser-Arg-Gly-Ser-Glu-Ser-Gly
Asp-Ser-Gly-Glu-Gly-Asp-Phe-Leu-Ala-Glu-Gly-Gly-Gly-Val-Arg
Gln-Phe-Thr-Ser-Ser-Thr-Ser-Tyr-Asn-Arg-Gly-Asp-Ser
Ser-Thr-Ser-Tyr-Asn-Arg-Gly-Asp-Ser-Thr-Phe-Glu-Ser-Lys-Ser
Lys-Glu-Ser-Ser-Ser-His-His-Pro-Gly-Ile-Ala-Glu-Phe-Pro-Ser
Tyr-Ser-Lys-Gln-Phe-Thr-Ser

Ser-Tyr-Ser-Lys-Gln-Phe-Thr-Ser-Ser-Thr-Ser-Tyr-Asn-Arg-Gly

The following peptides derive from Macrophage colony-stimulating factor 1 (CSF-1) (M-CSF) (MCSF) (Lanimostim) [Cleaved into: Processed macrophage colony-stimulating factor 1] (CSF1) (CSF1_HUMAN), these peptides are:

Ala-Glu-Pro-Glu-Gly-Gly-Pro-Ala-Ser-Glu-Gly-Ala-Ala-Arg-Pro-Leu-Pro-Arg
Ser-Glu-Gly-Ala-Ala-Arg-Pro-Leu-Pro-Arg

The following peptide derive from Endothelial cell-selective adhesion molecule (ESAM) (ESAM_HUMAN), this peptide is:
Thr-Thr-Asp-Gly-Ala-His-Pro-Gln-Pro-Ile

The following peptides derive from Thymosin beta-4 (T beta-4) (Fx) [Cleaved into: Hematopoietic system regulatory peptide (Seraspenide)] (TMSB4X) (TYB4_HUMAN), these peptides are:

Thr-Glu-Thr-Gln-Glu-Lys-Asn-Pro-Leu-Pro-Ser-Lys-Glu-Thr
Lys-Thr-Glu-Thr-Gln-Glu-Lys-Asn-Pro-Leu-Pro-Ser-Lys-Glu-Thr
Lys-Asn-Pro-Leu-Pro-Ser-Lys-Glu-Thr

The following peptide derive from Apolipoprotein A-IV (Apo-AIV) (ApoA-IV) (Apolipoprotein A4) (APOA4) (APOA4_HUMAN), this peptide is:
Thr-Gln-Val-Ser-Thr-Gln-Ala-Glu-Gln-Leu-Arg

The following peptide derive from Thymosin beta-4, Y-chromosomal (TMSB4Y) (TYB4Y_HUMAN), this peptide is:
Lys-Phe-Asp-Lys-Ser-Lys-Leu

The following peptide derive from Coagulation factor XIII A chain (Coagulation factor XIIIa) (EC 2.3.2.13) (Protein-glutamine gamma-glutamyltransferase A chain) (Transglutaminase A chain) (F13A1) (F13A_HUMAN), this peptide is:
Glu-Leu-Gln-Gly-Val-Val-Pro-Arg

The following peptides derive from Microtubule-associated protein RP/EB family member 1 (APC-binding protein EB1) (End-binding protein 1) (EB1) (MAPRE1) (MARE1 HUMAN), these peptides are

Thr-Ser-Ser-Ser-Ala-Ala-Pro-Gln-Arg-Pro-Ile
Lys-Lys-Pro-Leu-Thr-Ser-Ser-Ser-Ala-Ala-Pro-Gln-Arg-Pro-Ile-Ser-
Thr-Gln

The following peptide derive from Hepcidin (Liver-expressed antimicrobial peptide 1) (LEAP-1) (Putative liver tumor regressor) (PLTR) [Cleaved into: Hepcidin-25 (Hepc25); Hepcidin-20 (Hepc20)] (HAMP) (HEPC HUMAN), this peptide is:

Ser-Val-Phe-Pro-Gln-Gln-Thr-Gly-Gln-Leu-Ala-Glu-Leu-Gln-Pro-Gln-Asp-Arg-Ala-Gly-
Ala-Arg-Ala-Ser

The following peptide derive from Alpha-1-antitrypsin (Alpha-1 protease inhibitor) (Alpha-1-antiproteinase) (Serpin A1) [Cleaved into: Short peptide from AAT (SPAAT)] (SERPINA1) (A1AT_HUMAN), this peptide is:
Asn-Thr-Lys-Ser-Pro-Leu-Phe-Met-Gly-Lys-Val-Val-Asn-Pro-Thr-Gln-Lys

The following peptides derive from Alpha-2-macroglobulin (Alpha-2-M) (C3 and PZP-like alpha-2-macroglobulin domain-containing protein 5) (A2M) (A2MG_HUMAN), these peptides are:

Tyr-Lys-His-Tyr-Asp-Gly-Ser-Tyr
His-Thr-Glu-Ala-Gln-Ile-Gln-Glu-Glu-Gly-Thr-Val

The following peptide derive from Complement C3 (C3 and PZP-like alpha-2-macroglobulin domain-containing protein 1) [Cleaved into: Complement C3 beta chain; C3-beta-c (C3bc); Complement C3 alpha chain; C3a anaphylatoxin; Acylation stimulating protein (ASP) (C3adesArg); Complement C3b alpha' chain; Complement C3c alpha' chain fragment 1; Complement C3dg fragment; Complement C3g fragment; Complement C3d fragment; Complement C3f fragment; Complement C3c alpha' chain fragment 2] (C3) (CO3_HUMAN), this peptide is:
Arg-Ile-His-Trp-Glu-Ser-Ala-Ser

The following peptide derive from Collagen alpha-2(I) chain (Alpha-2 type I collagen) (COL1A2), Serglycin (Hematopoietic proteoglycan core protein) (Platelet proteoglycan core protein) (P.PG) (Secretory granule proteoglycan core protein) (SRGN) (CO1A2_HUMAN), this peptide is:
Val-Gly-Pro-Ala-Gly-Ala-Val-Gly-Pro-Arg

The following peptide derive from Fibrinogen beta chain [Cleaved into: Fibrinopeptide B; Fibrinogen beta chain] (FGB) (FIBB_HUMAN), this peptide is:
Asn-Asp-Asn-Glu-Glu-Gly-Phe-Phe

The following peptide derive from Caveolae-associated protein 2 (Cavin-2) (PS-p68) (Phosphatidylserine-binding protein) (Serum deprivation-response protein) (CAVIN2) (CAVN2_HUMAN), this peptide is:
Ala-Leu-Thr-Ser-Glu-Glu-Ala-Glu-Arg-Ser-Asp-Gly-Asp-Pro-Val-Gln-Pro-Ala

The following peptide derive from Apolipoprotein B-100 (Apo B-100) [Cleaved into: Apolipoprotein B-48 (Apo B-48)] (APOB) (APOB_HUMAN ), this peptide is:
Glu-Ile-Gln-Ile-Tyr-Lys-Lys-Leu-Arg

The following peptide derive from Latent-transforming growth factor beta-binding protein 2 (LTBP-2) (LTBP2) (LTBP2_HUMAN), this peptide is:
Thr-Pro-Leu-Gly-Gln-Gln-Gln-Pro-Ala-Pro-Arg

The following peptide derive from Complement C4-A (Acidic complement C4) (C3 and PZP-like alpha-2-macroglobulin domain-containing protein 2) [Cleaved into: Complement C4 beta chain; Complement C4-A alpha chain; C4a anaphylatoxin; C4b-A; C4d-A; Complement C4 gamma chain] (C4A) (CO4A_HUMAN), this peptide is:
Ala-Leu-Gln-Leu-Asn-Asn-Arg-Gln-Ile

The following peptide derive from Apolipoprotein C-II (Apo-CII) (ApoC-II) (Apolipoprotein C2) [Cleaved into: Proapolipoprotein C-II (ProapoC-II)] (APOC2) (APOC2_HUMAN), this peptide is:
Val-Leu-Ser-Val-Leu-Lys-Gly-Glu-Glu

The following peptide derive from Bridging integrator 2 (Breast cancer-associated protein 1) (BIN2) (BIN2 _HUMAN), this peptide is:
Ser-Leu-Val-Ile-Ser-Pro-Pro-Val-Arg-Thr-Ala-Thr-Val-Ser

The following peptide derive from Antithrombin-III (ATIII) (Serpin C1) (SERPINC1) (ANT3 _HUMAN), this peptide is:
Tyr-Val-Ser-Asp-Ala-Phe-His-Lys-Ala-Phe

The following peptides derive from Collagen alpha-1(III) chain (COL3A1) (CO3A1_HUMAN), these peptides are:

Gly-Ala-Asp-Gly-Val-Pro-Gly-Lys-Asp-Gly-Pro-Arg-Gly-Pro
Pro-Gly-Lys-Asp-Gly-Pro-Arg-Gly-Pro-Thr

The following peptide derive from Alpha-2-HS-glycoprotein (Alpha-2-Z-globulin) (Ba-alpha-2-glycoprotein) (Fetuin-A) [Cleaved into: Alpha-2-HS-glycoprotein chain A; Alpha-2-HS-glycoprotein chain B] (AHSG) (FETUA_HUMAN), this peptide is:
Ser-Leu-Gly-Ser-Pro-Ser-Gly-Glu-Val

The following peptide derive from Vitronectin (VN) (S-protein) (Serum-spreading factor) (V75) [Cleaved into: Vitronectin V65 subunit; Vitronectin V10 subunit; Somatomedin-B] (VTN) (VTNC _HUMAN), this peptide is:
Ser-Asp-Leu-Gln-Ala-Gln-Ser-Lys-Gly-Asn-Pro-Glu-Gln-Thr-Pro-Val

The following peptide derive from Tropomyosin alpha-3 chain (Gamma-tropomyosin) (Tropomyosin-3) (Tropomyosin-5) (hTM5) (TPM3) (TPM3_HUMAN), this peptide is:
Asp-Arg-Lys-Tyr-Glu-Glu-Val

The following peptide derive from Complement component C8 beta chain (Complement component 8 subunit beta) (C8B) (CO8B _HUMAN), this peptide is:
Leu-Gln-Arg-Val-Lys-Arg-Leu-Pro-Leu-Glu-Tyr-Ser-Tyr

The following peptide derive from Filamin-A (FLN-A) (Actin-binding protein 280) (ABP-280) (Alpha-filamin) (Endothelial actin-binding protein) (Filamin-1) (Non-muscle filamin) (FLNA) (FLNA_HUMAN), this peptide is:
Ser-Lys-Gly-Ala-Gly-Gly-Gln-Gly-Lys-Val-Ala-Ser-Lys-Ile-Val-Gly-Pro-Ser-Gly

The following peptides derive from Myosin-9 (Cellular myosin heavy chain, type A) (Myosin heavy chain 9) (Myosin heavy chain, non-muscle IIa) (Non-muscle myosin heavy chain A) (NMMHC-A) (Non-muscle myosin heavy chain IIa) (NMMHC II-a) (NMMHC-IIA) (MYH9) (MYH9 _HUMAN), these peptides are:

Glu-Glu-Glu-Leu-Val-Lys-Val-Arg-Glu-Lys-Gln-Leu-Ala
Glu-Glu-Gln-Leu-Glu-Glu-Glu-Glu-Ser-Ala-Arg-Gln

The following peptides derive from Adenylyl cyclase-associated protein 1 (CAP 1) (CAP1) (CAP1_HUMAN), these peptides are:

Ala-Gln-Ser-Gly-Pro-Val-Arg-Ser-Gly-Pro-Lys-Pro-Phe-Ser-Ala-Pro-Lys-Pro-Gln
Ala-Gln-Ser-Gly-Pro-Val-Arg-Ser-Gly-Pro-Lys-Pro-Phe-Ser-Ala-Pro-Lys-Pro-
Gln-Thr

The following peptide derive from Inter-alpha-trypsin inhibitor heavy chain H3 (ITI heavy chain H3) (ITI-HC3) (Inter-alpha-inhibitor heavy chain 3) (Serum-derived hyaluronan-associated protein) (SHAP) (ITIH3) (ITIH3_HUMAN), this peptide is:
Ala-Leu-Glu-Asn-His-Gly-Phe

The following peptides derive from Multimerin-1 (EMILIN-4) (Elastin microfibril interface located protein 4) (Elastin microfibril interfacer 4) (Endothelial cell multimerin) [Cleaved into: Platelet glycoprotein Ia*; 155 kDa platelet multimerin (p-155) (p155)] (MMRN1) (MMRN1_HUMAN), these peptides are:

Gly-Gly-Thr-Gly-Gly-Ile-Gly-Gly-Val-Gly-Gly-Thr-Gly-Gly-Val-Gly-Asn-Arg-
Ala-Pro-Arg
Val-Gly-Gly-Thr-Gly-Gly-Ile-Gly-Gly-Val-Gly-Gly-Thr-Gly-Gly-Val-Gly-Asn-
Arg-Ala-Pro-Arg

The following peptide derive from Plexin domain-containing protein 2 (Tumor endothelial marker 7-related protein) (PLXDC2) (PXDC2_HUMAN), this peptide is:
Asp-Thr-Asn-Arg-Ala-Ser-Val-Gly-Gln-Asp-Ser-Pro-Glu-Pro-Arg

The following peptide derive from Serglycin, Hematopoietic proteoglycan core protein, Platelet proteoglycan core protein, P.PG, Secretory granule proteoglycan core protein (SRGN_HUMAN) , this peptide is:
Asp-Arg-Asn-Leu-Pro-Ser-Asp-Ser-Gln-Asp-Leu-Gly-Gln-His-Gly

The following peptide derive from Zyxin, Zyxin-2 (ZYX) (ZYX_HUMAN), this peptide is:
Phe-Ser-Pro-Val-Thr-Pro-Lys-Phe-Thr-Pro-Val-Ala-Ser

AHSG (alpha-2-HS-glycoprotein/Alpha-2-Heremans-Schmid Glycoprotein/ fetuin-A. In our analysis indicates that AHSG [8] [9] has a central role and it connected several ICH stroke pathways. ITIH3 (Inter-alpha-trypsin inhibitor heavy chain H3) is a carrier of hyaluronan in serum, which is a major component of the extracellular matrix. In ICH stroke, due to hemorrhage, the veins and brain tissues/cells are ruptured that leading to injuries and wounds. As hyaluronan has role in wound healing [10] and the ITIH3 levels increase because ITIH3 has to carry as much of hyaluronan to the wound site in order to reduce damage to the brain tissue. C8B (Complement component C8 beta chain), COL1A2(Collagen alpha-2(I) chain, It is well known that due to stroke the extracellular matrix proteins such as fibronectin, collagen families, elastin, and others were overexpressed. Collagen, one of the main component of the extracellular matrix, and play a role in cell adhesion, migration and collagen remodeling. COL1A2(Collagen alpha-2(I) chain) fibrillar forming collagen. Another chain of collagen COL3A1(Collagen alpha-1(III) chain) were upregulated in the ICH stroke patients. Collagen type III occurs in most soft connective tissues along with type I collagen involved in regulation of cortical development. Type III Collagen levels [11] involves in the pathogenesis of saccular intracranial aneurysms. SERPINC1 (Antithrombin-III) is most important serine protease inhibitor in plasma that regulates the blood coagulation cascade. Antithrombin is a key endogenous anticoagulant that is necessary to control the clotting event during ICH stroke. In past studies demonstrated that SERPINC1 plasma levels in the whole cohort of patients during the acute event was significantly higher than that found in control groups (A new method to quantify β-antithrombin glycoform in plasma reveals increased [12] levels during the acute stroke event. In addition, Serpinc1/Antithrombin III showed a significant role [13] in the attenuation of salt-induced hypertension. Hypertension causes damage to the inner lining of the blood vessels and develops hemorrhagic stroke such as ICH. Altogether, the release of Serpinc1 peptides in the ICH stroke has a unique and significant indication to discriminate the ICH stoke from other strokes. Vitronectin (VN) is a multifunctional glycoprotein in the blood that leaks from the bloodstream into the injury site/extracellular matrix. The elevated level of vitronectin is considered a marker of Coronary Artery Disease (CAD) [14] or progression of CAD . Moreover, it also plays a role in vascular wound healing mechanisms due to ICH stroke in various brain parts. Particularly, it binds to platelet glycoproteins that facilitate platelet adhesion and subsequently aggregation at stroke site sites were vascular injury presents [15], APOA4 (Apolipoprotein A-IV) is involved in a various of physiological processes, however, platelet aggregation and inhibit the thrombosis that determines the ICH stroke-related the role [16], MMRN1(Multimerin-1) it supports platelet adhesion through mechanisms involving von Willebrand factor (VWF) and enhances platelet adhesion [17] to fibrillar collagen. Therefore, MMRN1 plays an important role via fibrillar collagens that synergistically enhance platelet adhesion at sites of vessel injury or thrombus formation due to the ICH stroke. CSF1 (Macrophage colony-stimulating factor 1) the patient having vascular damage to the old cerebral infarction that showed the elevated level of CSF than those control subjects . In our peptidomics analysis, we have observed that peptides generated by CSF1 are unique to ICH stroke as ICH patients have vascular damage, and that alters the CSF1 peptide levels.

The stroke, particularly ICH, directly impacts the cytoskeleton after the vein/vesicular injury/hemorrhage. However, mechanisms that result in cell/tissue injury are not fully understood. Cytoskeleton plays a crucial role such as maintaining the functional and structural integrity of cells, organelle transport, cell division, motility, and signaling. Therefore, cytoskeleton reflects the central to both cell health and stroke processes. The major component of the cytoskeleton are actin, filamin, myosin, tropomyosin, have a significant role in the integrity of cells. We have seen peptides from these proteins are significantly upregulated in ICH which indicates potential peptides marker for hemorrhage stroke. ACTB (Actin, cytoplasmic 1) Actin is a highly conserved protein that polymerizes to produce filaments that form cross-linked networks [18] in the cytoplasm of cells. Actin exists in both monomeric (G-actin) and polymeric (F-actin) forms, both forms playing key functions, [18] such as cell motility and contraction. Moreover, actin to maintains the integrity of neuronal processes by forming a rod between actine-cofilin [19] aggregate after the stroke. FLNA(Filamin-A) is a actin-binding proteins that play central role in platelet adhesion, aggregation and cytoskeleton remodeling. It anchors various transmembrane proteins to the actin cytoskeleton and serves as a scaffold for a wide range of cytoplasmic signaling proteins. Particularly FLNA interacts with several receptors GPIba and aIIbb3 integrin essential platelet [20] functioning after the hemorrage. After the vascular damage due to the ICH stroke, Filamin A (FLNA) is plays a significant role for cell-cell contact in vascular development, neuronal migration [21] and cardiac morphogenesis. MYH9(Myosin-9) Cellular myosin combined with actin appears to play a role in muscle contraction, cytokinesis, and specialized functions such as secretion and capping. It has shown in the role in the recovery of stroke, i.e. myosin e recovery stroke contributes to unidirectional stepping [22] of myosin along with actin. TPM3 (Tropomyosin alpha-3 chain), the cytoskeleton is mainly controlled by actin and a variety of acting binding proteins which also includes coiled-coil dimers of tropomyosin. It actively participates in various cellular events in muscle and non-muscle cells. For muscle contractions which is calcium-dependent regulation and the interaction of actin-containing thin filaments with myosin-containing thick filaments and in non-muscle cells multiple isoforms of tropomyosin expressed. Moreover, Smooth muscle contraction is regulated by interaction with caldesmon. The past report suggests that modification or mutation in tropomyosin leads to abnormal binding with both myosin cycling and calcium that lead to the [23] hypertrophic cardiomyopathy. CAP1(Adenylyl cyclase-associated protein 1) is involved in various cellular processes. Particularly, the remodeling of actin filaments, actin cytoskeleton, and its interactions with many actin-binding proteins. Moreover, it has direct or indirect interactions with cytoskeletal and extra cellular matrix proteins such as cofilin 1, βactin, profilin 1, destrin, actin depolymerizing factor, C-terminal binding protein matrix, [24] metalloproteinase 9, etc. Therefore, the ICH stroke unique peptides from CAP1 might serve as marker peptides.

Peptides are a microenvironment feature and have unrestricted access to facilities the pass-through cellular membrane, endothelial cell barrier, penetrate in to tissue, blood-brain barrier, junctions, etc. Therefore, serum peptides are most accurate to the physiological state of health than proteomics i.e., phenotypic. Even minute changes and unique signatures can be reflected in the peptidome (vitamins, cytokines, growth hormones are the peptides). This high susceptibility of serum peptidome to any pathophysiological disorders can be desirably reflected in peptidome is an advantage in biomarker discovery. our goal is to develop a multi-biomarker kit for differentiation of risk/susceptible group, early diagnosis, and diagnostic of intracranial hemorrhagic (ICH) stroke. To our knowledge, this is the first approach employed for serum peptidomics biomarker discovery of intracranial hemorrhagic (ICH) stroke. Furthermore, our workflows, including (sample preparation, MS measurements, and data analysis performance) are sensitive, and precise quantification facilitates the employing of our novel approach in various clinical applications including the diagnosis of intracranial hemorrhagic (ICH) stroke.

### References

[1] J. M. Wardlaw, V. Murray, E. Berge, and G. J. del Zoppo, "Thrombolysis for acute ischaemic stroke," Cochrane Database Syst. Rev., no. 7, 2014, doi: 10.1002/14651858.CD000213.pub3.
[2] A. I. Qureshi et al., "Acute Ischemic Stroke and COVID-19: An Analysis of 27 676 Patients," Stroke, vol. 52, no. 3, Art. no. 3, Mar. 2021, doi: 10.1161/STROKEAHA.120.031786.
[3] P. Belani et al., "COVID-19 Is an Independent Risk Factor for Acute Ischemic Stroke," AJNR Am. J. Neuroradiol., vol. 41, no. 8, pp. 1361-1364, Aug. 2020, doi: 10.3174/ajnr.A6650.
[4] R. Richter et al., "Composition of the peptide fraction in human blood plasma: database of circulating human peptides," J. Chromatogr. B. Biomed. Sci. App., vol. 726, no. 1-2, pp. 25-35, Apr. 1999, doi: 10.1016/s0378-4347(99)00012-2.
[5] H. Ay, "Biomarkers for stroke diagnosis," WO2014121252A1, Aug. 07, 2014 Accessed: Dec. 17, 2022. [Online]. Available: https://patents.google.com/patent/WO2014121252A1/en
[6] K. I. VADAKKAN, "Biomarkers for rapid detection of an occurrence of a stroke event," WO2014110674A1, Jul. 24, 2014 Accessed: Dec. 17, 2022. [Online]. Available: https://patents.google.com/patent/WO2014110674A1/en?oq=PCT%2fCA2014%2f05002 3
[7] J. M. Villalonga, A. S. ORIOL, and L. R. PASCUAL, "Biomarkers for stroke prognosis," WO2021009287A1, Jan. 21, 2021 Accessed: Dec. 17, 2022. [Online]. Available: https://patents.google.com/patent/WO2021009287A1/en?oq=PCT%2fEP2020%2f07015 2
[8] G. Li et al., "The impact of the AHSG genetic polymorphism on the risk of ischemic stroke: a case-control study," Int. J. Clin. Exp. Pathol., vol. 11, no. 10, pp. 5094-5100, 2018.
[9] S. Ma et al., "Association of AHSG gene polymorphisms with ischemic stroke in a Han Chinese population," Biochem. Genet., vol. 51, no. 11-12, pp. 916-926, Dec. 2013, doi: 10.1007/s 10528-013-9625-6.
[10] A. Shaharudin and Z. Aziz, "Effectiveness of hyaluronic acid and its derivatives on chronic wounds: a systematic review," J. Wound Care, vol. 25, no. 10, pp. 585-592, Oct. 2016, doi: 10.12968/jowc.2016.25.10.585.
[11] J. S. P. van den Berg et al., "Type III Collagen Deficiency in Saccular Intracranial Aneurysms," Stroke, vol. 30, no. 8, pp. 1628-1631, Aug. 1999, doi: 10.1161/01.STR.30.8.1628.
[12] M. E. de la Morena-Barrio et al., "A new method to quantify β-antithrombin glycoform in plasma reveals increased levels during the acute stroke event," Thromb. Res., vol. 136, no. 3, pp. 634-641, Sep. 2015, doi: 10.1016/j.thromres.2015.06.039.
[13] F. Wang et al., "Abstract P127: Serpinc1/Antithrombin III Attenuates Salt-Induced Hypertension," Hypertension, vol. 74, no. Suppl_1, pp. AP127-AP127, Sep. 2019, doi: 10.1161/hyp.74.suppl_1.P127.
[14] H. Ekmekci, H. Sonmez, O. B. Ekmekci, Z. Ozturk, N. Domanic, and E. Kokoglu, "Plasma vitronectin levels in patients with coronary atherosclerosis are increased and correlate with extent of disease," J. Thromb. Thrombolysis, vol. 14, no. 3, pp. 221-225, Dec. 2002, doi: 10.1023/a:1025000810466.
[15] P. Thiagarajan and K. L. Kelly, "Exposure of binding sites for vitronectin on platelets following stimulation," J. Biol. Chem., vol. 263, no. 6, pp. 3035-3038, Feb. 1988.
[16] X. R. Xu et al., "Apolipoprotein A-IV binds αIIbβ3 integrin and inhibits thrombosis," Nat. Commun., vol. 9, no. 1, p. 3608, Sep. 2018, doi: 10.1038/s41467-018-05806-0.
[17] A. Leatherdale et al., "Multimerin 1 supports platelet function in vivo and binds to specific GPAGPOGPX motifs in fibrillar collagens that enhance platelet adhesion," J. Thromb. Haemost. JTH, vol. 19, no. 2, pp. 547-561, Feb. 2021, doi: 10.1111/jth. 15171.
[18] A. Drazic et al., "NAA80 is actin's N-terminal acetyltransferase and regulates cytoskeleton assembly and cell motility," Proc. Natl. Acad. Sci. U. S. A., vol. 115, no. 17, pp. 4399-4404, Apr. 2018, doi: 10.1073/pnas.1718336115.
[19] S. J. Won et al., "Cofilin-actin rod formation in neuronal processes after brain ischemia," PLOS ONE, vol. 13, no. 10, p. e0198709, Oct. 2018, doi: 10.1371/journal.pone.0198709.
[20] J.-P. Rosa, H. Raslova, and M. Bryckaert, "Filamin A: key actor in platelet biology," Blood, vol. 134, no. 16, pp. 1279-1288, Oct. 2019, doi: 10.1182/blood.2019000014.
[21] Y. Feng et al., "Filamin A (FLNA) is required for cell-cell contact in vascular development and cardiac morphogenesis," Proc. Natl. Acad. Sci., vol. 103, no. 52, pp. 19836-19841, Dec. 2006, doi: 10.1073/pnas.0609628104.
[22] K. Shiroguchi, H. F. Chin, D. E. Hannemann, E. Muneyuki, E. M. D. L. Cruz, and K. K. Jr, "Direct Observation of the Myosin Va Recovery Stroke That Contributes to Unidirectional Stepping along Actin," PLOS Biol., vol. 9, no. 4, p. e1001031, Apr. 2011, doi: 10.1371/journal.pbio.1001031.
[23] A. Karibe et al., "Hypertrophic Cardiomyopathy Caused by a Novel α-Tropomyosin Mutation (V95A) Is Associated With Mild Cardiac Phenotype, Abnormal Calcium Binding to Troponin, Abnormal Myosin Cycling, and Poor Prognosis," Circulation, vol. 103, no. 1, pp. 65-71, Jan. 2001, doi: 10.1161/01.CIR.103.1.65.
[24] G. V. Kakurina, E. S. Kolegova, and I. V. Kondakova, "Adenylyl Cyclase-Associated Protein 1: Structure, Regulation, and Participation in Cellular Processes," Biochem. Biokhimiia, vol. 83, no. 1, pp. 45-53, Jan. 2018, doi: 10.1134/S0006297918010066.

## Claims

1. A method of *in vitro* diagnosing of intracranial hemorrhagic (ICH) stroke in human and differential diagnosing from acute ischemic stroke based on analysis sera levels of the following 5 quantitative peptides: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Arg-Ile-His-Trp-Glu-Ser-Ala-Ser, Ser-Tyr-Ser-Lys-Gln-Phe-Thr-Ser-Ser-Thr-Ser-Tyr-Asn-Arg-Gly are increased 16 folds or more in sera of ICH patients as compared with individuals without stroke and patients with acute ischemic stroke, wherein peptides are detected and measured in serum samples.

2. A method of *in vitro* diagnosing of intracranial hemorrhagic (ICH) stroke in human and differential diagnosing from acute ischemic stroke based on analysis sera levels of the following 7 quantitative peptides: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Lys-Asn-Pro-Leu-Pro-Ser-Lys-Glu-Thr,Ser-Tyr-Ser-Lys-Gln-Phe-Thr-Ser-Ser-Thr-Ser-Tyr-Asn-Arg-Gly are increased 10 folds or more in sera of ICH patients as compared with individuals without stroke and patients with acute ischemic stroke, wherein peptides are detected and measured in serum samples.

3. A method of *in vitro* diagnosing of intracranial hemorrhagic (ICH) stroke in human and differential diagnosing from acute ischemic stroke based on analysis sera levels of the following 10 quantitative peptides: Glu-Phe-Val-Ser-Glu-Thr-Glu-Ser-Arg-Gly-Ser-Glu, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Ala-Leu-Thr-Ser-Glu-Glu-Ala-Glu-Arg-Ser-Asp-Gly-Asp-Pro-Val-Gln-Pro-Ala, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Val-Leu-Ser-Val-Leu-Lys-Gly-Glu-Glu are increased 8 folds or more in sera of ICH patients as compared with individuals without stroke and in patients with acute ischemic stroke, wherein peptides are detected and measured in serum samples.

4. A method of *in vitro* diagnosing of intracranial hemorrhagic (ICH) stroke in human and differential diagnosing from acute ischemic stroke based on analysis sera levels of following 34 quantitative peptides: Glu-Phe-Val-Ser-Glu-Thr-Glu-Ser-Arg-Gly-Ser-Glu; Ala-Glu-Pro-Glu-Gly-Gly-Pro-Ala-Ser-Glu-Gly-Ala-Ala-Arg-Pro-Leu-Pro-Arg, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Phe-Val-Ser-Glu-Thr-Glu-Ser-Arg-Gly-Ser-Glu-Ser-Gly, Thr-Gln-Val-Ser-Thr-Gln-Ala-Glu-Gln-Leu-Arg, Lys-Phe-Asp-Lys-Ser-Lys-Leu, Glu-Leu-Gln-Gly-Val-Val-Pro-Arg, Thr-Ser-Ser-Ser-Ala-Ala-Pro-Gln-Arg-Pro-Ile, Ser-Val-Phe-Pro-Gln-Gln-Thr-Gly-Gln-Leu-Ala-Glu-Leu-Gln-Pro-Gln-Asp-Arg-Ala-Gly-Ala-Arg-Ala-Ser, Asn-Thr-Lys-Ser-Pro-Leu-Phe-Met-Gly-Lys-Val-Val-Asn-Pro-Thr-Gln-Lys, Tyr-Lys-His-Tyr-Asp-Gly-Ser-Tyr, Asp-Thr-Asn-Arg-Ala-Ser-Val-Gly-Gln-Asp-Ser-Pro-Glu-Pro-Arg, Phe-Ser-Pro-Val-Thr-Pro-Lys-Phe-Thr-Pro-Val-Ala-Ser, Asp-Ser-Gly-Glu-Gly-Asp-Phe-Leu-Ala-Glu-Gly-Gly-Gly-Val-Arg, Gln-Phe-Thr-Ser-Ser-Thr-Ser-Tyr-Asn-Arg-Gly-Asp-Ser, Arg-Ile-His-Trp-Glu-Ser-Ala-Ser, Val-Gly-Pro-Ala-Gly-Ala-Val-Gly-Pro-Arg, Asp-Arg-Asn-Leu-Pro-Ser-Asp-Ser-Gln-Asp-Leu-Gly-Gln-His-Gly, Lys-Lys-Pro-Leu-Thr-Ser-Ser-Ser-Ala-Ala-Pro-Gln-Arg-Pro-Ile-Ser-Thr-Gln, Asn-Asp-Asn-Glu-Glu-Gly-Phe-Phe, Ala-Leu-Thr-Ser-Glu-Glu-Ala-Glu-Arg-Ser-Asp-Gly-Asp-Pro-Val-Gln-Pro-Ala, His-Thr-Glu-Ala-Gln-Ile-Gln-Glu-Glu-Gly-Thr-Val, Ser-Thr-Ser-Tyr-Asn-Arg-Gly-Asp-Ser-Thr-Phe-Glu-Ser-Lys-Ser, Glu-Ile-Gln-Ile-Tyr-Lys-Lys-Leu-Arg, Lys-Glu-Ser-Ser-Ser-His-His-Pro-Gly-Ile-Ala-Glu-Phe-Pro-Ser, Thr-Pro-Leu-Gly-Gln-Gln-Gln-Pro-Ala-Pro-Arg, Tyr-Ser-Lys-Gln-Phe-Thr-Ser, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Ala-Leu-Gln-Leu-Asn-Asn-Arg-Gln-Ile, Val-Leu-Ser-Val-Leu-Lys-Gly-Glu-Glu, Ser-Leu-Val-Ile-Ser-Pro-Pro-Val-Arg-Thr-Ala-Thr-Val-Ser, wherein the serum levels of the mentioned 34 peptides in sera of patients with ICH are increased 2 folds or more as compared with individuals without stroke and patients with acute ischemic stroke and a list of the following 16 qualitative peptides: Tyr-Val-Ser-Asp-Ala-Phe-His-Lys-Ala-Phe, Gly-Ala-Asp-Gly-Val-Pro-Gly-Lys-Asp-Gly-Pro-Arg-Gly-Pro, Pro-Gly-Lys-Asp-Gly-Pro-Arg-Gly-Pro-Thr, Ser-Leu-Gly-Ser-Pro-Ser-Gly-Glu-Val, Ser-Asp-Leu-Gln-Ala-Gln-Ser-Lys-Gly-Asn-Pro-Glu-Gln-Thr-Pro-Val, Asp-Arg-Lys-Tyr-Glu-Glu-Val, Leu-Gln-Arg-Val-Lys-Arg-Leu-Pro-Leu-Glu-Tyr-Ser-Tyr, Ser-Glu-Gly-Ala-Ala-Arg-Pro-Leu-Pro-Arg, Ser-Lys-Gly-Ala-Gly-Gly-Gln-Gly-Lys-Val-Ala-Ser-Lys-Ile-Val-Gly-Pro-Ser-Gly, Glu-Glu-Glu-Leu-Val-Lys-Val-Arg-Glu-Lys-Gln-Leu-Ala, Glu-Glu-Gln-Leu-Glu-Glu-Glu-Glu-Ser-Ala-Arg-Gln, Ala-Gln-Ser-Gly-Pro-Val-Arg-Ser-Gly-Pro-Lys-Pro-Phe-Ser-Ala-Pro-Lys-Pro-Gln, Ala-Gln-Ser-Gly-Pro-Val-Arg-Ser-Gly-Pro-Lys-Pro-Phe-Ser-Ala-Pro-Lys-Pro-Gln-Thr, Ala-Leu-Glu-Asn-His-Gly-Phe, Gly-Gly-Thr-Gly-Gly-Ile-Gly-Gly-Val-Gly-Gly-Thr-Gly-Gly-Val-Gly-Asn-Arg-Ala-Pro-Arg, Val-Gly-Gly-Thr-Gly-Gly-Ile-Gly-Gly-Val-Gly-Gly-Thr-Gly-Gly-Val-Gly-Asn-Arg-Ala-Pro-Arg wherein mentioned 16 peptides are detectable in sera of the patients with ICH compared to serum samples from the individuals without stroke and patients with acute ischemic stroke. wherein peptides are detected and measured in serum samples.

5. The method according to any claims 1-4, wherein the serum peptides are measured by tandem mass spectrometry.

## Patentansprüche

1. Ein Verfahren zur Diagnose *in vitro* des intrakraniellen hämorrhagischen (ICH) Schlaganfalls beim Menschen und zur Differenzialdiagnose des akuten ischämischen Schlaganfall , basierend auf der Grundlage der Analyse der Serumspiegel der folgenden 5 quantitativen Peptide: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Arg-Ile-His-Trp-Glu-Ser-Ala-Ser, Ser-Tyr-Ser-Lys-Gln-Phe-Thr-Ser-Ser-Thr-Ser-Tyr-Asn-Arg-Gly, die in Seren von ICH-Patienten im Vergleich zu Personen ohne Schlaganfall und Patienten mit akutem ischämischem Schlaganfall um das 16-fache oder mehr erhöht sind, wobei Peptide in Serumproben nachgewiesen und gemessen werden.

2. Eine Methode zur Diagnose *in vitro* von intrakraniellen hämorrhagischen (ICH) Schlaganfällen beim Menschen und zur Differenzialdiagnose von akuten ischämischen Schlaganfällen, basierend auf der Analyse der Serumspiegel der folgenden 7 quantitativen Peptide: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Lys-Asn-Pro-Leu-Pro-Ser-Lys-Glu-Thr,Ser-Tyr-Ser-Lys-Gln-Phe-Thr-Ser-Ser-Thr-Ser-Tyr-Asn-Arg-Gly die in Seren von ICH-Patienten im Vergleich zu Personen ohne Schlaganfall und Patienten mit akutem ischämischem Schlaganfall um das 10-fache oder mehr erhöht sind, wobei Peptide in Serumproben nachgewiesen und gemessen werden.

3. Eine Methode zur Diagnose *in vitro* von intrakraniellen hämorrhagischen (ICH) Schlaganfällen beim Menschen und zur Differenzialdiagnose von akuten ischämischen Schlaganfällen, basierend auf der Analyse der Serumspiegel der folgenden 10 quantitativen Peptide: Glu-Phe-Val-Ser-Glu-Thr-Glu-Ser-Arg-Gly-Ser-Glu, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Ala-Leu-Thr-Ser-Glu-Glu-Ala-Glu-Arg-Ser-Asp-Gly-Asp-Pro-Val-Gln-Pro-Ala, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Val-Leu-Ser-Val-Leu-Lys-Gly-Glu-Glu die in Seren von ICH-Patienten im Vergleich zu Personen ohne Schlaganfall und zu Patienten mit akutem ischämischem Schlaganfall um das 8-fache oder mehr erhöht sind, wobei Peptide in Serumproben nachgewiesen und gemessen werden.

4. Eine Methode zur Diagnose *in vitro* von intrakraniellen hämorrhagischen (ICH) Schlaganfällen beim Menschen und zur Differentialdiagnose von akuten ischämischen Schlaganfällen, basierend auf der Analyse der Serumspiegel der folgenden 34 quantitativen Peptide: Glu-Phe-Val-Ser-Glu-Thr-Glu-Ser-Arg-Gly-Ser-Glu; Ala-Glu-Pro-Glu-Gly-Gly-Pro-Ala-Ser-Glu-Gly-Ala-Ala-Arg-Pro-Leu-Pro-Arg, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Phe-Val-Ser-Glu-Thr-Glu-Ser-Arg-Gly-Ser-Glu-Ser-Gly, Thr-Gln-Val-Ser-Thr-Gln-Ala-Glu-Gln-Leu-Arg, Lys-Phe-Asp-Lys-Ser-Lys-Leu, Glu-Leu-Gln-Gly-Val-Val-Pro-Arg, Thr-Ser-Ser-Ser-Ala-Ala-Pro-Gln-Arg-Pro-Ile, Ser-Val-Phe-Pro-Gln-Gln-Thr-Gly-Gln-Leu-Ala-Glu-Leu-Gln-Pro-Gln-Asp-Arg-Ala-Gly-Ala-Arg-Ala-Ser, Asn-Thr-Lys-Ser-Pro-Leu-Phe-Met-Gly-Lys-Val-Val-Asn-Pro-Thr-Gln-Lys, Tyr-Lys-His-Tyr-Asp-Gly-Ser-Tyr, Asp-Thr-Asn-Arg-Ala-Ser-Val-Gly-Gln-Asp-Ser-Pro-Glu-Pro-Arg, Phe-Ser-Pro-Val-Thr-Pro-Lys-Phe-Thr-Pro-Val-Ala-Ser, Asp-Ser-Gly-Glu-Gly-Asp-Phe-Leu-Ala-Glu-Gly-Gly-Gly-Val-Arg, Gln-Phe-Thr-Ser-Ser-Thr-Ser-Tyr-Asn-Arg-Gly-Asp-Ser, Arg-Ile-His-Trp-Glu-Ser-Ala-Ser, Val-Gly-Pro-Ala-Gly-Ala-Val-Gly-Pro-Arg, Asp-Arg-Asn-Leu-Pro-Ser-Asp-Ser-Gln-Asp-Leu-Gly-Gln-His-Gly, Lys-Lys-Pro-Leu-Thr-Ser-Ser-Ser-Ala-Ala-Pro-Gln-Arg-Pro-Ile-Ser-Thr-Gln, Asn-Asp-Asn-Glu-Glu-Gly-Phe-Phe, Ala-Leu-Thr-Ser-Glu-Glu-Ala-Glu-Arg-Ser-Asp-Gly-Asp-Pro-Val-Gln-Pro-Ala, His-Thr-Glu-Ala-Gln-Ile-Gln-Glu-Glu-Gly-Thr-Val, Ser-Thr-Ser-Tyr-Asn-Arg-Gly-Asp-Ser-Thr-Phe-Glu-Ser-Lys-Ser, Glu-Ile-Gln-Ile-Tyr-Lys-Lys-Leu-Arg, Lys-Glu-Ser-Ser-Ser-His-His-Pro-Gly-Ile-Ala-Glu-Phe-Pro-Ser, Thr-Pro-Leu-Gly-Gln-Gln-Gln-Pro-Ala-Pro-Arg, Tyr-Ser-Lys-Gln-Phe-Thr-Ser, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Ala-Leu-Gln-Leu-Asn-Asn-Arg-Gln-Ile, Val-Leu-Ser-Val-Leu-Lys-Gly-Glu-Glu, Ser-Leu-Val-Ile-Ser-Pro-Pro-Val-Arg-Thr-Ala-Thr-Val-Ser, wobei die Serumspiegel der erwähnten 34 Peptide in den Seren von Patienten mit ICH im Vergleich zu Personen ohne Schlaganfall und Patienten mit akutem ischämischem Schlaganfall um das 2-fache oder mehr erhöht sind, und eine Liste der folgenden 16 qualitativen Peptide: Tyr-Val-Ser-Asp-Ala-Phe-His-Lys-Ala-Phe, Gly-Ala-Asp-Gly-Val-Pro-Gly-Lys-Asp-Gly-Pro-Arg-Gly-Pro, Pro-Gly-Lys-Asp-Gly-Pro-Arg-Gly-Pro-Thr, Ser-Leu-Gly-Ser-Pro-Ser-Gly-Glu-Val, Ser-Asp-Leu-Gln-Ala-Gln-Ser-Lys-Gly-Asn-Pro-Glu-Gln-Thr-Pro-Val, Asp-Arg-Lys-Tyr-Glu-Glu-Val, Leu-Gln-Arg-Val-Lys-Arg-Leu-Pro-Leu-Glu-Tyr-Ser-Tyr, Ser-Glu-Gly-Ala-Ala-Arg-Pro-Leu-Pro-Arg, Ser-Lys-Gly-Ala-Gly-Gly-Gln-Gly-Lys-Val-Ala-Ser-Lys-Ile-Val-Gly-Pro-Ser-Gly, Glu-Glu-Glu-Leu-Val-Lys-Val-Arg-Glu-Lys-Gln-Leu-Ala, Glu-Glu-Gln-Leu-Glu-Glu-Glu-Glu-Ser-Ala-Arg-Gln, Ala-Gln-Ser-Gly-Pro-Val-Arg-Ser-Gly-Pro-Lys-Pro-Phe-Ser-Ala-Pro-Lys-Pro-Gln, Ala-Gln-Ser-Gly-Pro-Val-Arg-Ser-Gly-Pro-Lys-Pro-Phe-Ser-Ala-Pro-Lys-Pro-Gln-Thr, Ala-Leu-Glu-Asn-His-Gly-Phe, Gly-Gly-Thr-Gly-Gly-Ile-Gly-Gly-Val-Gly-Gly-Thr-Gly-Gly-Val-Gly-Asn-Arg-Ala-Pro-Arg, Val-Gly-Gly-Thr-Gly-Gly-Ile-Gly-Gly-Val-Gly-Gly-Thr-Gly-Gly-Val-Gly-Asn-Arg-Ala-Pro-Arg wobei die erwähnten 16 Peptide in Seren von Patienten mit ICH nachweisbar sind, verglichen mit Serumproben von Personen ohne Schlaganfall und Patienten mit akutem ischämischem Schlaganfall, wobei Peptide in Serumproben nachgewiesen und gemessen werden.

5. Verfahren nach einem der Ansprüche 1-4, wobei die Serumpeptide durch Tandem-Massenspektrometrie gemessen werden.

## Revendications

1. Une méthode de diagnostic *in vitro* de l'accident vasculaire cérébral hémorragique intracrânien (ICH) chez l'homme et de diagnostic différentiel par rapport à l'accident vasculaire cérébral ischémique aigu basée sur l'analyse des niveaux de sérum des 5 peptides quantitatifs suivants: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Arg-Ile-His-Trp-Glu-Ser-Ala-Ser, Ser-Tyr-Ser-Lys-Gln-Phe-Thr-Ser-Ser-Thr-Ser-Tyr-Asn-Arg-Gly sont multipliés par 16 ou plus dans les sérums des patients atteints d'HIC par rapport aux individus sans accident vasculaire cérébral et aux patients atteints d'accident vasculaire cérébral ischémique aigu, les peptides étant détectés et mesurés dans les échantillons de sérum.

2. Une méthode de diagnostic *in vitro* de l'accident vasculaire cérébral hémorragique intracrânien (ICH) chez l'homme et de diagnostic différentiel par rapport à l'accident vasculaire cérébral ischémique aigu, basée sur l'analyse des niveaux de sérum des 7 peptides quantitatifs suivants: Glu-Phe-Val-Ser-Glu-Thr-Glu-Ser-Arg-Gly-Ser-Glu, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Lys-Asn-Pro-Leu-Pro-Ser-Lys-Glu-Thr,Ser-Tyr-Ser-Lys-Gln-Phe-Thr-Ser-Ser-Thr-Ser-Tyr-Asn-Arg-Gly sont multipliés par 10 ou plus dans le sérum des patients atteints d'HIC par rapport aux personnes n'ayant pas subi d'accident vasculaire cérébral et aux patients ayant subi un accident vasculaire cérébral ischémique aigu, les peptides étant détectés et mesurés dans les échantillons de sérum.

3. Une méthode de diagnostic *in vitro* de l'accident vasculaire cérébral hémorragique intracrânien (ICH) chez l'homme et de diagnostic différentiel par rapport à l'accident vasculaire cérébral ischémique aigu, basée sur l'analyse des niveaux de sérum des 10 peptides quantitatifs suivants: Glu-Phe-Val-Ser-Glu-Thr-Glu-Ser-Arg-Gly-Ser-Glu, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Asp-Thr-Asn-Arg-Ala-Ser-Val-Gly-Gln-Asp-Ser-Pro-Glu-Pro-Arg, Arg-Ile-His-Trp-Glu-Ser-Ala-Ser, Ala-Leu-Thr-Ser-Glu-Glu-Ala-Glu-Arg-Ser-Asp-Gly-Asp-Pro-Val-Gln-Pro-Ala, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Val-Leu-Ser-Val-Leu-Lys-Gly-Glu-Glu sont multipliés par 8 ou plus dans le sérum des patients atteints d'HIC par rapport aux personnes n'ayant pas subi d'accident vasculaire cérébral et aux patients ayant subi un accident vasculaire cérébral ischémique aigu, les peptides étant détectés et mesurés dans les échantillons de sérum.

4. Une méthode de diagnostic *in vitro* de l'accident vasculaire cérébral hémorragique intracrânien (ICH) chez l'homme et de diagnostic différentiel par rapport à l'accident vasculaire cérébral ischémique aigu, basée sur l'analyse des niveaux de sérum des 34 peptides quantitatifs suivants: Glu-Phe-Val-Ser-Glu-Thr-Glu-Ser-Arg-Gly-Ser-Glu; Ala-Glu-Pro-Glu-Gly-Gly-Pro-Ala-Ser-Glu-Gly-Ala-Ala-Arg-Pro-Leu-Pro-Arg, Thr-Thr-Asp-Gly-Ala-His-Pro-Gln-Pro-Ile, Thr-Glu-Thr-Gln-Glu-Lys-Asn-Pro-Leu-Pro-Ser-Lys-Glu-Thr, Lys-Thr-Glu-Thr-Gln-Glu-Lys-Asn-Pro-Leu-Pro-Ser-Lys-Glu-Thr, Phe-Val-Ser-Glu-Thr-Glu-Ser-Arg-Gly-Ser-Glu-Ser-Gly, Thr-Gln-Val-Ser-Thr-Gln-Ala-Glu-Gln-Leu-Arg, Lys-Phe-Asp-Lys-Ser-Lys-Leu, Glu-Leu-Gln-Gly-Val-Val-Pro-Arg, Thr-Ser-Ser-Ser-Ala-Ala-Pro-Gln-Arg-Pro-Ile, Ser-Val-Phe-Pro-Gln-Gln-Thr-Gly-Gln-Leu-Ala-Glu-Leu-Gln-Pro-Gln-Asp-Arg-Ala-Gly-Ala-Arg-Ala-Ser, Asn-Thr-Lys-Ser-Pro-Leu-Phe-Met-Gly-Lys-Val-Val-Asn-Pro-Thr-Gln-Lys, Tyr-Lys-His-Tyr-Asp-Gly-Ser-Tyr, Asp-Thr-Asn-Arg-Ala-Ser-Val-Gly-Gln-Asp-Ser-Pro-Glu-Pro-Arg, Phe-Ser-Pro-Val-Thr-Pro-Lys-Phe-Thr-Pro-Val-Ala-Ser, Asp-Ser-Gly-Glu-Gly-Asp-Phe-Leu-Ala-Glu-Gly-Gly-Gly-Val-Arg, Gln-Phe-Thr-Ser-Ser-Thr-Ser-Tyr-Asn-Arg-Gly-Asp-Ser, Arg-Ile-His-Trp-Glu-Ser-Ala-Ser, Val-Gly-Pro-Ala-Gly-Ala-Val-Gly-Pro-Arg, Asp-Arg-Asn-Leu-Pro-Ser-Asp-Ser-Gln-Asp-Leu-Gly-Gln-His-Gly, Lys-Lys-Pro-Leu-Thr-Ser-Ser-Ser-Ala-Ala-Pro-Gln-Arg-Pro-Ile-Ser-Thr-Gln, Asn-Asp-Asn-Glu-Glu-Gly-Phe-Phe, Ala-Leu-Thr-Ser-Glu-Glu-Ala-Glu-Arg-Ser-Asp-Gly-Asp-Pro-Val-Gln-Pro-Ala, His-Thr-Glu-Ala-Gln-Ile-Gln-Glu-Glu-Gly-Thr-Val, Ser-Thr-Ser-Tyr-Asn-Arg-Gly-Asp-Ser-Thr-Phe-Glu-Ser-Lys-Ser, Glu-Ile-Gln-Ile-Tyr-Lys-Lys-Leu-Arg, Lys-Glu-Ser-Ser-Ser-His-His-Pro-Gly-Ile-Ala-Glu-Phe-Pro-Ser, Thr-Pro-Leu-Gly-Gln-Gln-Gln-Pro-Ala-Pro-Arg, Tyr-Ser-Lys-Gln-Phe-Thr-Ser, Lys-Asn-Pro-Leu-Pro-Ser-Lys-Glu-Thr, Ser-Tyr-Ser-Lys-Gln-Phe-Thr-Ser-Ser-Thr-Ser-Tyr-Asn-Arg-Gly, Ala-Leu-Gln-Leu-Asn-Asn-Arg-Gln-Ile, Val-Leu-Ser-Val-Leu-Lys-Gly-Glu-Glu, Ser-Leu-Val-Ile-Ser-Pro-Pro-Val-Arg-Thr-Ala-Thr-Val-Ser, dans lequel les niveaux sériques des 34 peptides mentionnés dans les sérums de patients atteints d'HIC sont multipliés par 2 ou plus par rapport aux individus n'ayant pas subi d'AVC et aux patients ayant subi un AVC ischémique aigu, et une liste des 16 peptides qualitatifs suivants: Tyr-Val-Ser-Asp-Ala-Phe-His-Lys-Ala-Phe, Gly-Ala-Asp-Gly-Val-Pro-Gly-Lys-Asp-Gly-Pro-Arg-Gly-Pro, Pro-Gly-Lys-Asp-Gly-Pro-Arg-Gly-Pro-Thr, Ser-Leu-Gly-Ser-Pro-Ser-Gly-Glu-Val, Ser-Asp-Leu-Gln-Ala-Gln-Ser-Lys-Gly-Asn-Pro-Glu-Gln-Thr-Pro-Val, Asp-Arg-Lys-Tyr-Glu-Glu-Val, Leu-Gln-Arg-Val-Lys-Arg-Leu-Pro-Leu-Glu-Tyr-Ser-Tyr, Ser-Glu-Gly-Ala-Ala-Arg-Pro-Leu-Pro-Arg, Ser-Lys-Gly-Ala-Gly-Gly-Gln-Gly-Lys-Val-Ala-Ser-Lys-Ile-Val-Gly-Pro-Ser-Gly, Glu-Glu-Glu-Leu-Val-Lys-Val-Arg-Glu-Lys-Gln-Leu-Ala, Glu-Glu-Gln-Leu-Glu-Glu-Glu-Glu-Ser-Ala-Arg-Gln, Ala-Gln-Ser-Gly-Pro-Val-Arg-Ser-Gly-Pro-Lys-Pro-Phe-Ser-Ala-Pro-Lys-Pro-Gln, Ala-Gln-Ser-Gly-Pro-Val-Arg-Ser-Gly-Pro-Lys-Pro-Phe-Ser-Ala-Pro-Lys-Pro-Gln-Thr, Ala-Leu-Glu-Asn-His-Gly-Phe, Gly-Gly-Thr-Gly-Gly-Ile-Gly-Gly-Val-Gly-Gly-Thr-Gly-Gly-Val-Gly-Asn-Arg-Ala-Pro-Arg, Val-Gly-Gly-Thr-Gly-Gly-Ile-Gly-Gly-Val-Gly-Gly-Thr-Gly-Gly-Val-Gly-Asn-Arg-Ala-Pro-Arg dans lequel les 16 peptides mentionnés sont détectables dans le sérum des patients atteints d'HIC par rapport aux échantillons de sérum des personnes n'ayant pas subi d'accident vasculaire cérébral et des patients ayant subi un accident ischémique cérébral aigu. dans lequel les peptides sont détectés et mesurés dans les échantillons de sérum.

5. La méthode selon l'une des réclamations 1 à 4, dans laquelle les peptides sériques sont mesurés par spectrométrie de masse en tandem.
